# EUROPEAN PATENT APPLICATION

(11) **EP 4 667 569 A1**
(43) Date of publication of application: **24.12.2025**
(21) Application number: 24756987.4
(22) Date of filing: 16.02.2024
(51) Int. Cl.: C12N 15/11, A61K 31/712, A61K 31/7105, A61K 47/59, A61K 48/00, A61P 21/00, A61P 25/02, A61P 25/14, A61P 25/16, A61P 25/28, A61P 43/00, C07H 21/02

(54) **MULTIVALENT BINDING NUCLEIC ACID AGENT**

(30) Priority: 16.02.2023 JP 2023022549
(71) Applicant: INSTITUTE OF SCIENCE TOKYO, Tokyo 152-8550 (JP)
(72) Inventor: YOKOTA, Takanori, Tokyo 152-8550 (JP); SAKAUE, Fumika, Tokyo 152-8550 (JP); HARA, Rintaro, Tokyo 152-8550 (JP); MIURA, Motoki, Tokyo 152-8550 (JP)
(74) Representative: Mewburn Ellis LLP
(86) International application number: PCT/JP2024/005438
(87) International publication number: WO 2024/172148

(57) **Abstract**

An object of the present invention is to provide a novel nucleic acid agent capable of efficiently suppressing the aggregation of TDP-43 protein.

It provides a multivalent binding nucleic acid agent which binds to TDP-43 protein or a fragment thereof, comprising two or more nucleic acid strands, wherein each of the two or more nucleic acid strands comprises a TDP-43 binding sequence capable of binding to TDP-43 protein or a fragment thereof, and wherein: (1) the two or more nucleic acid strands are bound via a linker, or (2) the two or more nucleic acid strands comprise an adapter sequence that enables formation of multiplex strands between the two or more nucleic acid strands.

## Description

### Technical Field

The present invention relates to a multivalent binding nucleic acid agent which binds to TDP-43 protein or a fragment thereof, and a pharmaceutical composition for preventing or treating neurodegenerative diseases, and the like.

### Background Art

Amyotrophic lateral sclerosis (ALS) is an incurable neurological disease in which motor neurons degenerate, causing muscle weakness throughout the body. The main symptoms thereof include muscle atrophy and muscle weakness. As the disease progresses, functional disorders of the limbs and the trunk, gait disturbance, and, in the nervous system, dysarthria and swallowing disorders, and the like appear, leading to respiratory disorders due to paralysis of the respiratory muscles.

It is known that RNA-binding protein such as TDP-43 protein is associated with the pathology of ALS. In particular, while about 90% of TDP-43 protein is localized in the nucleus in normal neurons, most of TDP-43 protein accumulates in cytoplasmic inclusion bodies in degenerated neurons of ALS and disappears from the nucleus. Therefore, it is considered that, in the degenerated neurons of ALS, a TDP-43 protein aggregate exhibits its toxicity in the cytoplasm and simultaneously loses its function in the nucleus.

Previous studies using transgenic mouse have revealed that the overexpression of wild-type human TDP-43 protein leads to the degeneration of motor neurons and the appearance of symptoms similar to amyotrophic lateral sclerosis. Similarly, when mutant TDP-43 protein found in hereditary amyotrophic lateral sclerosis was expressed, symptoms similar to amyotrophic lateral sclerosis were observed. It is considered that these results suggest that the overexpression of TDP-43 protein causes toxicity in motor neurons and the like, regardless of whether TDP-43 protein is wild-type TDP-43 protein or mutant-TDP-43 protein.

Patent Literature 1 and 2 disclose, based on the fact that TDP-43 protein is RNA-binding protein, a UGGAA repeat sequence and a UG repeat sequence were found as a base sequence which binds to TDP-43 protein, and bait nucleic acid comprising these repeat sequences is effective in inhibiting the aggregation of TDP-43 protein.

To further enhance the aggregation suppression effect against TDP-43 protein based on high affinity binding sequences, novel technologies are required.

### Citation List

### Patent Literature

Patent Literature 1: WO2016/088797
Patent Literature 2: WO2020/027311

### Summary of Invention

### Technical Problem

An object of the present invention is to provide a novel nucleic acid agent capable of efficiently suppressing the aggregation of TDP-43 protein.

### Solution to Problem

To solve the above-described problems, the present inventors have developed a divalent binding nucleic acid agent in which a spacer capable of controlling the interaction between TDP-43 proteins is introduced between two TDP-43 binding sequences. The divalent binding nucleic acid agent of the present invention can efficiently suppress the aggregate formation of TDP-43 protein based on the spacer positioned between the TDP-43 binding sequences, and exhibits a noticeable effect of suppressing the aggregation of even mutant TDP-43 protein which is prone to aggregation because it cannot form a dimer. Further, it has been revealed that the two TDP-43 binding sequences constituting the nucleic acid agent of the present invention cooperate with each other to assist TDP-43 protein to form a dimer and that this cooperative action is amplified by the presence of the spacer introduced between the TDP-43 binding sequences.

The present invention is based on the above-described findings, and provides the following.
(1) A multivalent binding nucleic acid agent which binds to TDP-43 protein or a fragment thereof, comprising two or more nucleic acid strands, wherein each of the two or more nucleic acid strands comprises a TDP-43 binding sequence capable of binding to TDP-43 protein or a fragment thereof, and wherein:
   (a) the two or more nucleic acid strands are bound via a linker, or
   (b) the two or more nucleic acid strands comprise an adapter sequence that enables formation of multiplex strands between the two or more nucleic acid strands.
(2) The nucleic acid agent of (1), wherein the linker has a rigidity and/or a length that can reduce an aggregate formation by two or more monomeric TDP-43 proteins which are bound to the TDP-43 binding sequences of the two or more nucleic acid strands.
(3) The nucleic acid agent of (2), wherein the reduction of the aggregate formation is based on a reduced binding between the C-terminal regions of the TDP-43 proteins and/or an increased phase separation thereof.
(4) The nucleic acid agent of any one of (1) to (3), wherein the linker comprises a nucleic acid, peptide, polyether group, and/or hydrocarbon group.
(5) The nucleic acid agent of (4), wherein the nucleic acid consists of one or two to 50 nucleosides linked via internucleoside bonds.
(6) The nucleic acid agent of (5), wherein the nucleoside comprises a natural nucleoside and/or a non-natural nucleoside.
(7) The nucleic acid agent of (4), wherein the nucleic acid is a peptide nucleic acid.
(8) The nucleic acid agent of (4), wherein the polyether group is a polyethylene glycol group.
(9) The nucleic acid agent of (4), wherein the hydrocarbon group is an optionally substituted C₂₋₃₀ hydrocarbon group.
(10) The nucleic acid agent of any one of (1) to (9), wherein the linker comprises a region consisting of a single-stranded nucleic acid.
(11) The nucleic acid agent of (10), wherein the region consisting of the single-stranded nucleic acid comprises a hairpin structure.
(12) The nucleic acid agent of (10) or (11), further comprising a complementary strand which comprises a base sequence complementary to at least part of the region consisting of the single-stranded nucleic acid, wherein the region consisting of the single-stranded nucleic acid and the complementary strand form a multiplex strand structure.
(13) The nucleic acid agent of (12), wherein the region consisting of the single-stranded nucleic acid comprises a non-complementary base, and/or an insertion sequence and/or a deletion of one or more bases, with respect to the complementary strand.
(14) The nucleic acid agent of (13), wherein the region consisting of the single-stranded nucleic acid comprises one to three of the non-complementary bases.
(15) The nucleic acid agent of (13), wherein the insertion sequence consists of one to eight bases.
(16) The nucleic acid agent of (13), wherein the deletion consists of one to four consecutive bases.
(17) The nucleic acid agent of (12 to 16), wherein the complementary strand comprises a natural nucleoside and/or a non-natural nucleoside.
(18) The nucleic acid agent of (1), wherein the adapter sequence is 8 to 50 bases in length.
(19) The nucleic acid agent of any one of (1) to (18), wherein the TDP-43 binding sequence binds to an RNA recognition motif (RRM) of the TDP-43 protein.
(20) The nucleic acid agent of any one of (1) to (19), wherein the TDP-43 binding sequence consists of a repeat sequence represented by the following formula (I):

   (X₀ G X₁ X₂ X₃ ... Xₘ)ₙ (I),

   wherein X₀ is T or U; X₁, X₂, X₃, ..., and Xₘ are independently any of A, C, G, T, or U, and may be identical or different, or X₁, X₂, X₃, ..., and Xₘ are absent; m is 1 to 10; and n represents a repeat number of 2 to 50.
(21) The nucleic acid agent of any one of (1) to (19), wherein the TDP-43 binding sequence consists of a repeat sequence represented by the following formula (IV):

   (X G)ₙ , (IV)

   wherein X represents T or U, and n is three or more.
(22) The nucleic acid agent of (21), wherein each of the TDP-43 binding sequence can bind to one TDP-43 protein or a fragment thereof.
(23) The nucleic acid agent of (22), wherein n is 6 or less.
(24) The nucleic acid agent of any one of (21) to (23), wherein the repeat sequence comprises a natural ribonucleoside and/or a non-natural ribonucleoside.
(25) The nucleic acid agent of (24), wherein the non-natural ribonucleoside is 2'-O-methyl-modified nucleoside, 2'-O-methoxyethyl-modified nucleoside, or 2'-O-[2-(N-methylcarbamoyl)ethyl]-modified nucleoside.
(26) The nucleic acid agent of any one of (1) to (25) for reducing aggregation of the TDP-43 protein and/or the fragment thereof.
(27) A pharmaceutical composition comprising the nucleic acid agent of any one of (1) to (26) as an active ingredient.
(28) The pharmaceutical composition of (27) for preventing or treating a neurodegenerative disease.
(29) The pharmaceutical composition of (28), wherein the neurodegenerative disease is TDP-43 proteinopathy.
(30) The pharmaceutical composition of (28) or (29), wherein the neurodegenerative disease is selected from the group consisting of amyotrophic lateral sclerosis (ALS) which is sporadic or familial, frontotemporal lobar degeneration (FTLD), Alzheimer's disease, dementia with Lewy bodies, Huntington's disease, Parkinson's disease, argyrophilic grain dementia (Grene's disease), Perry syndrome, progressive supranuclear palsy, corticobasal degeneration, and multiple system atrophy.

The contents of the disclosures in Japanese Patent Application No. 2023-022549 which form the basis for priority of the present application are incorporated herein.

### Advantageous Effects of Invention

The present invention provides a multivalent binding nucleic acid agent capable of efficiently suppressing the aggregation of TDP-43 protein.

### Brief Description of Drawing

[Figure 1] Figure 1 shows the structures of various bait nucleic acids. Figure 1A shows monovalent bait nucleic acid comprising one TDP-43 binding sequence and divalent bait nucleic acid comprising two TDP-43 binding sequences and no spacer, which were used as controls. FIG. 1B shows two TDP-43 binding sequences and divalent bait nucleic acid comprising a spacer consisting of nucleic acid between the TDP-43 binding sequences.

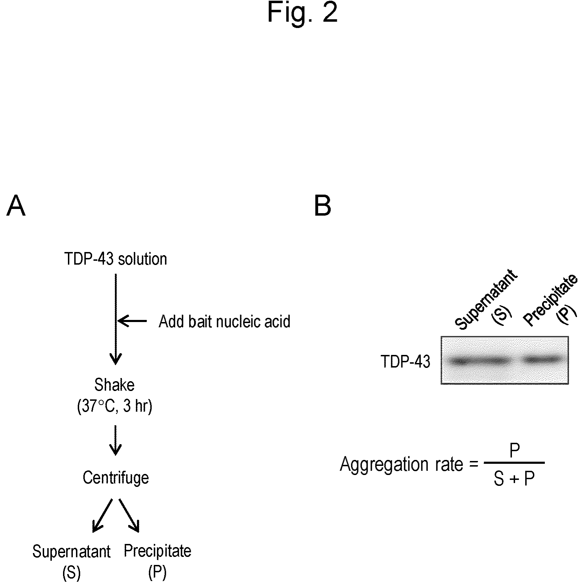

[Figure 2] Figure 2 shows a method for evaluating the aggregation rate of TDP-43 protein. Figure 2A shows steps of adding bait nucleic acid to a TDP-43 protein solution, and then separating the resulting solution into a precipitated fraction comprising aggregated TDP-43 protein and a supernatant fraction comprising non-aggregated TDP-43 protein. Figure 2B shows a method for calculating the aggregation rate from the measured amount of the TDP-43 protein in each fraction, the amount being obtained by measuring the amount of the TDP-43 protein in the supernatant fraction (S) and the precipitated fraction (P).
[Figure 3] Figure 3 shows an aggregation inhibitory effect of various bait nucleic acids against TDP-43 protein. Figure 3A shows the results of Western blotting. For each bait nucleic acid, "S" indicates the supernatant fraction and "P" indicates a lane loaded with the precipitated fraction. Figure 3B shows the aggregation rate of TDP-43 protein when various bait nucleic acids were used. The error bar indicates a standard error.
[Figure 4] Figure 4 shows the aggregation suppression effect against 6M-mutant TDP-43 protein which does not form a dimer. The error bar indicates a standard error. ***: P < 0.001, **: P < 0.01, *: P < 0.05, ns: not significant (Tukey's multiple comparison test).
[Figure 5] Figure 5 shows the results of detecting a monomer and a dimer of each of wild-type TDP-43 protein and 6M-mutant TDP-43 protein in the presence of various bait nucleic acids.
[Figure 6] Figure 6 shows the results of quantifying the ratio of dimers to monomers from the band intensities in the Western blotting shown in Figure 5. Figure 6A shows the results of wild-type TDP-43 protein. Figure 6B shows the results of 6M-mutant TDP-43 protein.
[Figure 7] Figure 7 shows divalent bait nucleic acids comprising triethylene glycol (TEG), hexaethylene glycol (HEG), or a C₁₂ alkylene chain as a spacer.
[Figure 8] Figure 8 shows the aggregation inhibitory effect of various bait nucleic acids against TDP-43 protein. The error bar indicates a standard error.
[Figure 9] Figure 9 shows the structures of various bait nucleic acids. Figure 9A shows the structures of divalent bait nucleic acids A₁₂, A₂₄, and A₄₈. Figure 9B shows the structure of A₁₂ trivalent which is trivalent bait nucleic acid.
[Figure 10] Figure 10 shows the structures of various bait nucleic acids. Figure 10A shows the structures of divalent bait nucleic acids A₁₂/t₁₂, A₂₄/t₂₄, and A₄₈/t₄₈. Figure 10B shows the structure of A₁₂/t₁₂ trivalent which is trivalent bait nucleic acid.
[Figure 11] Figure 11 shows the structures of divalent bait nucleic acid complexes Adapter1/C1 and Adapter2/C2.

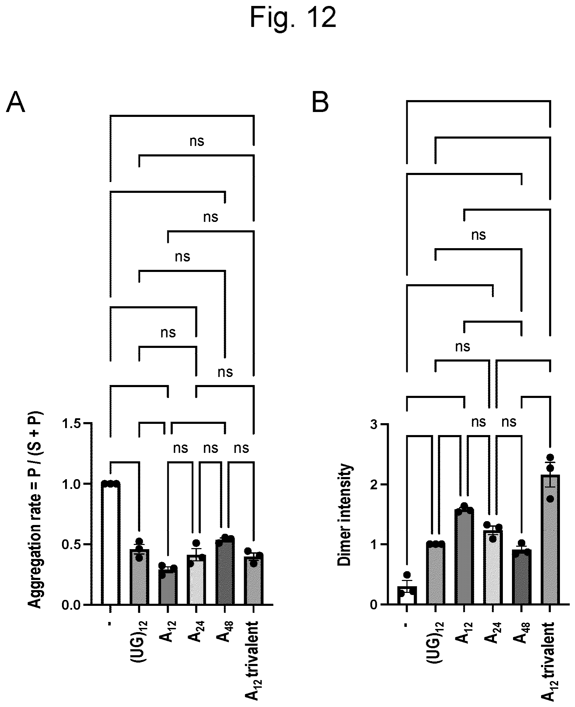

[Figure 12] Figure 12 shows the results of performing an in vitro aggregation assay and the quantification of a dimer. Figure 12A shows the aggregation inhibitory effect of various bait nucleic acids against TDP-43 protein. Figure 12B shows the results of quantifying a dimer of wild-type TDP-43 protein in the presence of various bait nucleic acids. The error bar indicates a standard error. A significant difference was detected between two groups not showing "ns" (P < 0.05), but a significant difference was not detected between two groups showing "ns" (Tukey's multiple comparison test; one-way ANOVA).
[Figure 13] Figure 13 shows the results of performing an in vitro aggregation assay and the quantification of a dimer. Figure 13A shows the aggregation inhibitory effect of various bait nucleic acids against TDP-43 protein. Figure 13B shows the results of quantifying a dimer of wild-type TDP-43 protein in the presence of various bait nucleic acids. The error bar indicates a standard error.
[Figure 14] Figure 14 shows the correlation between the quantitative value of a dimer and the aggregation rate in the in vitro aggregation assay.
[Figure 15] Figure 15 shows the structures of various bait nucleic acids.
[Figure 16] Figure 16 shows the structures of various bait nucleic acids.

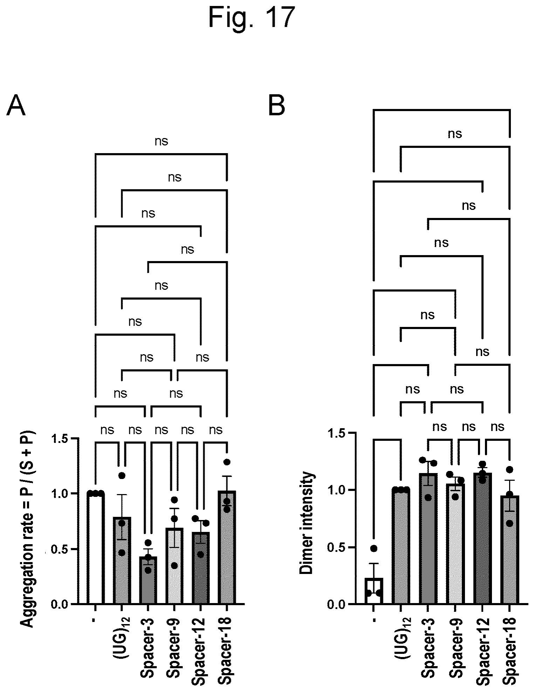

[Figure 17] Figure 17 shows the results of performing an in vitro aggregation assay and the quantification of a dimer. Figure 17A shows the aggregation inhibitory effect of various bait nucleic acids against TDP-43 protein. Figure 17B shows the results of quantifying a dimer of wild-type TDP-43 protein in the presence of various bait nucleic acids. The error bar indicates a standard error. A significant difference was detected between two groups not showing "ns" (P < 0.05), but a significant difference was not detected between two groups showing "ns" (Tukey's multiple comparison test; one-way ANOVA).

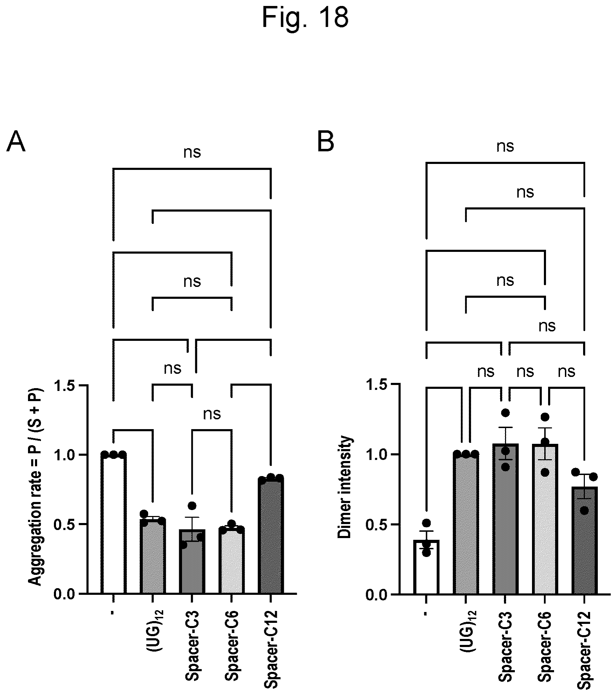

[Figure 18] Figure 18 shows the results of performing an in vitro aggregation assay and the quantification of a dimer. Figure 18A shows the aggregation inhibitory effect of various bait nucleic acids against TDP-43 protein. Figure 18B shows the results of quantifying a dimer of wild-type TDP-43 protein in the presence of various bait nucleic acids. The error bar indicates a standard error. A significant difference was detected between two groups not showing "ns" (P < 0.05), but a significant difference was not detected between two groups showing "ns" (Tukey's multiple comparison test; one-way ANOVA).

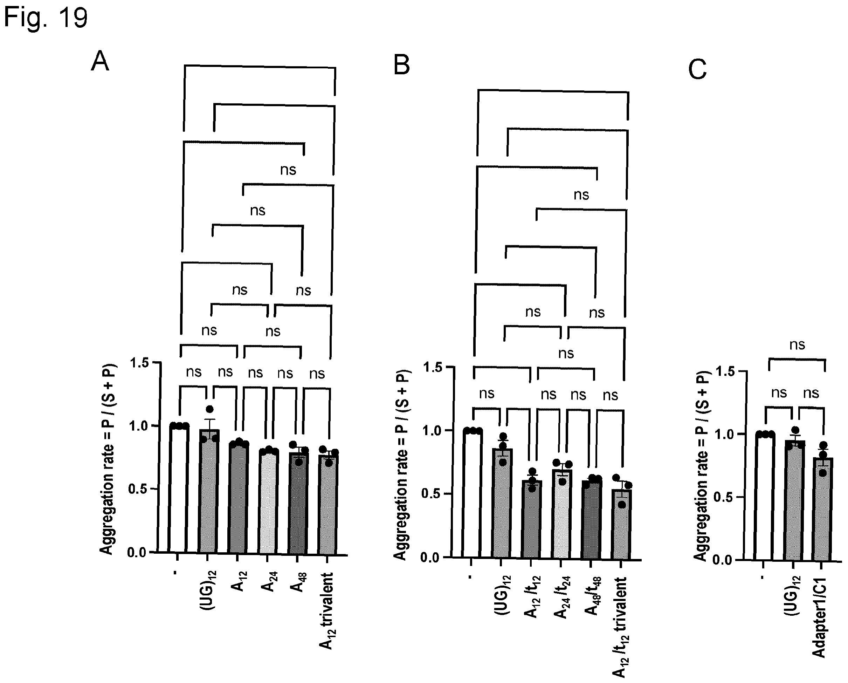

[Figure 19] Figure 19 shows the aggregation inhibitory effect of various bait nucleic acids or bait nucleic acid complexes against 6M-mutant TDP-43 protein. Figure 19A shows the structures of divalent bait nucleic acids A₁₂, A₂₄, A₄₈, and A₁₂ trivalent. Figure 19B shows the structures of bait nucleic acids A₁₂/t₁₂, A₂₄/t₂₄, A₄₈/t₄₈, and A₁₂/t₁₂ trivalent. Figure 19C shows the result of a bait nucleic acid complex Adapter1/C1. The error bar indicates a standard error. A significant difference was detected between two groups not showing "ns" (P < 0.05), but a significant difference was not detected between two groups showing "ns" (Tukey's multiple comparison test; one-way ANOVA).

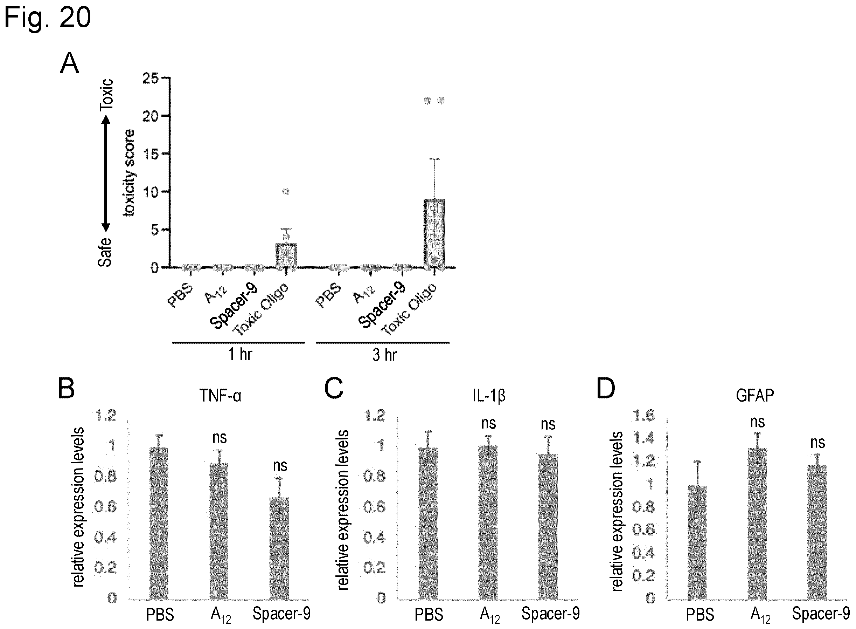

[Figure 20] Figure 20 shows the results of evaluating the toxicity of bait nucleic acids. Figure 20A shows the results of evaluating acute toxicity. Figure 20B shows the results of quantifying the relative expression level of an inflammatory marker of TNF-α mRNA. Figure 20C shows the results of quantifying the relative expression level of an inflammatory marker of IL-1β mRNA. Figure 20D shows the results of quantifying the relative expression level of a gliosis marker of GFAP mRNA. The error bar indicates a standard error. "ns" indicates that a significant difference was not detected for a PBS-treated group (Tukey's multiple comparison test; one-way ANOVA).
[Figure 21] Figure 21 shows the results of analyzing the survival rates of ALS model mice to which a vehicle (PBS) or bait nucleic acid A₁₂ or Spacer-9 was intraventricularly administered.
[Figure 22] Figure 22 shows the results of evaluating, by a rotarod test, the motor function of the ALS model mice to which a vehicle (PBS) or bait nucleic acid A₁₂ or Spacer-9 was intraventricularly administered.

### Description of Embodiments

### 1. Multivalent binding nucleic acid agent

### 1-1. Overview

The first aspect of the present invention is a multivalent binding nucleic acid agent which binds to TDP-43 protein or a fragment thereof. The multivalent binding nucleic acid agent of the present invention comprises two or more nucleic acid strands comprising a TDP-43 binding sequence capable of binding to TDP-43 protein or a fragment thereof. The multivalent binding nucleic acid agent of the present invention can suppress the aggregation of TDP-43 protein and stabilize a TDP-43 protein dimer.

### 1-2. Definition of terms

The "aggregation" of polypeptide means herein the gathering of two or more of the same or different types of polypeptides. This specification refers to an assembly of polypeptides aggregated to form a mass as "aggregate". In general, when polypeptide is denatured, hydrophobic amino acids buried inside the three-dimensional structure are exposed to the polypeptide surface, so that polypeptide molecules are likely to associate with each other. As a result, a plurality of polypeptide molecules assemble to form a large aggregate in many cases. Accumulation of the polypeptide aggregate damages cells, so that the cellular function can decrease in some cases.

"TDP-43 protein (Transactivation responsive region (TAR) DNA-binding protein 43)" means herein protein which belongs to the hnRNP family and binds to RNA to play important RNA metabolism functions such as RNA splicing, miRNA synthesis, and RNA transport. TDP-43 protein comprises, from the N-terminal side, an N-terminal domain, an RNA recognition motif 1 (RRM1), an RNA recognition motif 2 (RRM2), and a glycine-rich domain. In general, it is known that TDP-43 protein forms a dimer and exhibits normal functions in the nucleus, while the monomer is more likely to aggregate than the dimer and is associated with pathological conditions, and it is known that TDP-43 protein is localized in the cytoplasm with an increased proportion of the monomer in degenerated neurons of ALS, and exhibits molecular pathologies such as aggregate formation, fragmentation, and phosphorylation. Different regions in TDP-43 protein are important for the two types of interactions of the transformation of TDP-43 protein into a dimer and the aggregation of monomers. More specifically, the transformation of TDP-43 protein into a dimer is mediated by binding of the N-terminal domains. In contrast thereto, in the aggregation of monomers, the glycine-rich domain positioned on the C-terminal side forms the core of the aggregate. However, it has also been reported that weak multivalent interaction in the C-terminal region promotes the liquid-liquid phase separation (LLPS) and maintains liquidity (Grese, X. R., et al., EMBO Rep, 2021, 22, e53632). A familial mutation of a TDP-43 gene occurs primarily in the glycine-rich domain, and the occurrence of the mutation increases the aggregation properties of TDP-43 protein. However, the overexpression of TDP-43 protein, regardless of whether TDP-43 protein is wild-type TDP-43 protein or mutant- TDP-43 protein, results in intracytoplasmic aggregation, exhibiting toxicity, and the overexpression in neurons leads to neurodegeneration, causing neurodegenerative diseases. A disease caused by the degeneration or aggregation of TDP-43 protein or a disease involving the degeneration or aggregation of TDP-43 protein is referred to as "TDP-43 proteinopathy ".

The "fragment thereof" of TDP-43 protein means herein any polypeptide fragment or any peptide fragment that comprises a part of TDP-43 protein. Examples thereof include fragments that have aggregation properties as in full-length TDP-43 protein. Specific examples thereof include fragments comprising an RNA recognition motif (RRM) and/or a glycine-rich domain, a C-terminal fragment, and the like. The "C-terminal fragment " of TDP-43 protein refers to a fragment comprising the C-terminal side region of TDP-43 protein. Examples thereof include a fragment positioned on the C-terminal side of TDP-43 protein and comprising the glycine-rich domain that enables formation of the core of the aggregate. It is known that, in patients with sporadic amyotrophic lateral sclerosis (ALS) and patients with some of familial amyotrophic lateral sclerosis, C-terminal fragments having a molecular weight of 25 to 35 kDa derived from TDP-43 protein are found in brain tissues and are involved in the pathology. This C-terminal fragment has aggregation properties as in full-length TDP-43 protein, and can aggregate together with full-length TDP-43 protein and exhibit toxicity.

The "suppressing aggregation" or "suppressing aggregate formation" of TDP-43 protein or a fragment thereof means herein not only 100% suppression but 75% or more, 50% or more, 20% or more, or 10% or more suppression as compared to a case where a nucleic acid agent such as bait nucleic acid is not introduced. The aggregation level of TDP-43 protein or a fragment thereof can be evaluated by, for example, mixing a nucleic acid agent with TDP-43 protein or a fragment thereof, shaking the mixture as necessary, detecting the amount of TDP-43 protein or a fragment thereof for each of a non-aggregated fraction and an aggregated fraction obtained by separation by sedimentation, centrifugation, filtration, and the like, and calculating the ratio of the TDP-43 protein or a fragment thereof comprised in the aggregated fraction as the aggregation rate (Figure 2).

The "stabilizing a TDP-43 protein dimer" means herein that the ratio of dimers to monomers is improved as compared to a case where a nucleic acid agent such as bait nucleic acid is not introduced. Thus, the ratio of the dimer may not only be stabilized up to 100%, but may be maintained at 75% or more, 50% or more, 20% or more, or 10% or more. The ratio of the dimers to the monomers can be evaluated by, for example, mixing a nucleic acid agent with TDP-43 protein or a fragment thereof, shaking the mixture as necessary, carrying out a bridging reaction using a bridging agent such as glutaraldehyde, separating the monomer and the dimer based on their molecular weight using Western blotting or the like for detection, and calculating the ratio of the dimers to the sum of the monomers and the dimers.

The "TDP-43 binding sequence" means herein any nucleic acid sequence, any nucleoside sequence, or any base sequence capable of binding to TDP-43 protein or a fragment thereof. The TDP-43 binding sequence may be a sequence which binds to any region of TDP-43 protein or may be either a sequence which binds to an RNA recognition motif (RRM) of TDP-43 protein or a sequence which binds to a region other than the RRM. The sequences which bind to the RRM of TDP-43 protein are known. As the TDP-43 binding sequence, a known sequence may be used or a sequence capable of binding to TDP-43 protein or the RRM thereof can be specified by a binding assay such as a gel shift assay. In addition, an aptamer (nucleic acid aptamer) which binds to TDP-43 protein can also be used as the TDP-43 binding sequence.

The "multiplex strand" herein refers to a complex or a structure formed by two or more nucleic acid strands bound to each other. Examples thereof include a duplex strand formed by hybridization of two nucleic acid strands, a triplex strand formed by binding of three nucleic acid strands, and the like.

Herein, "bait nucleic acid" refers to a nucleic acid that specifically binds to a particular target molecule in a living body and modifies a function of the target molecule. A target that interacts with a bait is also referred to as a "prey".

The term "nucleic acid" or "nucleic acid molecule" used herein may refer to a nucleotide or nucleoside of a monomer, and may mean an oligonucleotide consisting of a plurality of monomers, or means a polynucleotide in the case of a polymer. A "natural nucleic acid" refers to a nucleic acid that exists in nature. Examples of the natural nucleic acid include a natural nucleoside, natural nucleotide, and the like, as described below. A "non-natural nucleic acid" or "artificial nucleic acid" refers to any nucleic acid other than a natural nucleic acid. Examples of the non-natural nucleic acid or the artificial nucleic acid include a non-natural nucleoside, non-natural nucleotide, and the like, as described below.

A "nucleic acid strand" or simply "strand" herein means two or more nucleosides linked via an internucleoside bond, and may be, e.g., an oligonucleotide or a polynucleotide. A full-length strand or a partial length strand of a nucleic acid strand can be produced, e.g., by a chemical synthesis using an automated synthesizer, or by an enzymatic step using a polymerase, a ligase, or a restricted reaction. A nucleic acid strand may comprise a natural nucleotide and/or a non-natural nucleotide.

A "nucleoside" generally means a molecule consisting of a combination of a base and a sugar. The sugar moiety of a nucleoside is usually, but not limited to, composed of pentofuranosyl sugar, and specific examples thereof include ribose and deoxyribose. The base moiety of nucleoside (nucleobase) is usually a heterocyclic base moiety. Without limitation, examples thereof include adenine, cytosine, guanine, thymine, or uracil as well as other modified nucleobases (modified bases).

A "nucleotide" refers to a molecule in which a phosphate group is covalently bound to the sugar moiety of the nucleoside. In the case of a nucleotide comprising pentofuranosyl sugar, a phosphate group is usually linked to a hydroxyl group at the 2', 3', or 5' position of the sugar.

An "oligonucleotide" refers to a linear oligomer formed by linking several to dozens of neighboring nucleotides through a covalent bond between a hydroxyl group and a phosphate group in the sugar moiety. Furthermore, a "polynucleotide" refers to a linear polymer formed by linking with covalent bonds dozens or more, preferably hundreds or more of nucleotides, namely more nucleotides than in an oligonucleotide. It is considered that the phosphate group generally forms an internucleoside bond inside the structure of an oligonucleotide or a polynucleotide.

A "natural nucleoside" refers herein to a nucleoside that exists in nature. Examples thereof include a ribonucleoside consisting of a ribose and the aforementioned base such as adenine, cytosine, guanine, or uracil, or a deoxyribonucleoside consisting of a deoxyribose and the aforementioned base such as adenine, cytosine, guanine, or thymine. In this regard, a ribonucleoside found in RNA, and a deoxyribonucleoside found in DNA are herein often referred to as "DNA nucleoside" and "RNA nucleoside", respectively.

A "natural nucleotide" means herein a nucleotide that exists in nature, namely a molecule in which a phosphate group is covalently bound to the sugar moiety of the aforementioned natural nucleoside. Examples thereof include a ribonucleotide which is known as a constituent of RNA, and in which a phosphate group is bound to a ribonucleoside, and a deoxyribonucleotide, which is known as a constituent of DNA, and in which a phosphate group is bound to a deoxyribonucleoside.

A "non-natural nucleotide" means herein any nucleotide other than a natural nucleotide. For example, it comprises a modified nucleotide and a nucleotide mimic. A "modified nucleotide" means herein a nucleotide having any one or more of a modified sugar moiety, a modified internucleoside bond, and a modified nucleobase. The term "nucleotide mimic" herein comprises a structure used to substitute a nucleoside and a linkage at one or more positions in an oligomer compound. Examples of the nucleotide mimic comprise a peptide nucleic acid, and a morpholino nucleic acid (morpholino linked with -N(H)-C(=O)-O- or other non-phosphodiester linkages). The peptide nucleic acid (PNA) is a nucleotide mimic having a main chain in which N-(2-aminoethyl)glycine in place of a sugar is linked with an amide linkage. A nucleic acid strand comprising a non-natural oligonucleotide herein has in many cases preferable properties such as enhanced cellular uptake, enhanced affinity for a target nucleic acid, increased stability in the presence of a nuclease, and an increase in inhibitory activity. Therefore, it is more preferable than a natural nucleotide.

A "non-natural nucleoside" means herein any nucleoside other than a natural nucleoside. For example, it comprises a modified nucleoside and a nucleoside mimic. A "modified nucleoside" means herein a nucleoside having a modified sugar moiety and/or a modified nucleobase.

A "mimic" refers herein to a functional group that substitutes a sugar, a nucleobase, and/or an internucleoside bond. In general, a mimic is used in place of a sugar or a combination of a sugar-internucleoside bond, and a nucleobase is maintained for hybridization to a target to be selected. The term "nucleoside mimic" used herein comprises a structure to be used for substituting a sugar at one or more positions of an oligomer compound, or substituting a sugar and a base, or substituting a bond between monomer subunits constituting an oligomer compound. An "oligomer compound" means a polymer composed of linked monomer subunits that can at least hybridize to a region of a nucleic acid molecule. Examples of a nucleoside mimic comprise a morpholino, cyclohexenyl, cyclohexyl, tetrahydropyranyl, bicyclic or tricyclic sugar mimic such as a nucleoside mimic having a non-furanose sugar unit.

A "modified sugar" refers to a sugar in which a natural sugar moiety (i.e., sugar moiety found in DNA(2'-H) or RNA(2'-OH)) has undergone a substitution and/or any change. "Sugar modification" refers to substitution and/or any change from a natural sugar moiety. A nucleic acid strand may comprise in some cases one or more modified nucleosides comprising a modified sugar. A "sugar-modified nucleoside" means a nucleoside having a modified sugar moiety. Such a sugar-modified nucleoside can confer a beneficial biological property such as enhanced nuclease stability, increased binding affinity, or the like to a nucleic acid strand. In a specific embodiment, a nucleoside comprises a chemically modified ribofuranose ring moiety. Examples of a chemically modified ribofuranose ring comprise, but are not limited to, addition of a substituent (comprising 5' and 2' substituents), formation of a bicyclic nucleic acid (bridged nucleic acid, BNA) through bridge formation of a non-geminal ring atom, substitution of a ribosyl ring oxygen atom with S, N(R), or C(R1)(R2) (wherein R, R1, and R2 independently represent H, a C₁-C₁₂ alkyl, or a protecting group), and a combination thereof.

Examples of the sugar-modified nucleoside comprise, but are not limited to, a nucleoside comprising a substituent such as 5'-vinyl, 5'-methyl (R or S), 5'-allyl (R or S), 4'-S, 2'-F(2'-fluorogroup), 2'-OCH₃ (2'-O-Me group or 2'-O-methyl group), 2'-O-[2-(*N-*methylcarbamoyl)ethyl] (2'-O-MCE group), and 2'-O-methoxyethyl (2'-O-MOE or 2-O(CH₂)₂OCH₃). A substituent at the 2' position may be selected from allyl, amino, azide, thio, -O-allyl, -O-C₁-C₁₀ alkyl, -OCF₃, -O(CH₂)₂SCH₃, -O(CH₂)₂-O-N(Rm)(Rn), and O-CH₂-C(=O)-N(Rm)(Rn), wherein Rm and Rn are independently H or a substituted or unsubstituted C₁-C₁₀ alkyl. A "2'-modified sugar" means a furanosyl sugar modified at the 2' position. A nucleoside comprising a 2'-modified sugar may be referred to as a "2'-modified nucleoside" or a "2'-sugar-modified nucleoside".

A "bicyclic nucleoside" refers to a modified nucleoside comprising a bicyclic sugar moiety. A nucleic acid comprising a bicyclic sugar moiety is commonly referred to as bridged nucleic acid (BNA). A nucleoside comprising a bicyclic sugar moiety is sometimes referred to as a "bridged nucleoside", "bridged-type non-natural nucleoside", or "BNA nucleoside".

A bicyclic sugar may be a sugar in which the carbon atom at the 2' position and the carbon atom at the 4' position are bridged with two or more atoms. Examples of a bicyclic sugar are known to those skilled in the art. A subgroup of nucleic acids comprising a bicyclic sugar (BNA) or of BNA nucleosides may be described as having a carbon atom at the 2' position and a carbon atom at the 4' position which are bridged with 4'-(CH₂)ₚ-O-2', 4'-(CH₂)ₚ-CH₂-2', 4'-(CH₂)ₚ-S-2', 4'-(CH₂)ₚ-OCO-2', 4'-(CH₂)ₙ-N(R₃)-O-(CH₂)ₘ-2' [wherein p, m, and n represent integers from 1 to 4, from 0 to 2, and from 1 to 3, respectively; and R₃ represents a hydrogen atom, an alkyl group, an alkenyl group, a cycloalkyl group, an aryl group, an aralkyl group, an acyl group, a sulfonyl group, and a unit substituent (e.g., a fluorescently or chemiluminescently labeled molecule, a functional group with nucleic acid cleavage activity, and an intracellular or nuclear localization signal peptide)]. Furthermore, with respect to a BNA or a BNA nucleoside in a certain embodiment, in the OR₂ substituent of the carbon atom at the 3' position and the OR₁ substituent of the carbon atom at the 5' position, R₁ and R₂ are typically hydrogen atoms, but may be the same or different from each other, or may also be a protecting group for a hydroxyl group for nucleic acid synthesis, an alkyl group, an alkenyl group, a cycloalkyl group, an aryl group, an aralkyl group, an acyl group, a sulfonyl group, a silyl group, a phosphate group, a phosphate group protected by a protecting group for nucleic acid synthesis, or P(R₄)R₅ [wherein R₄ and R₅, may be the same or different from each other, and respectively represent a hydroxyl group, a hydroxyl group protected by a protecting group for nucleic acid synthesis, a mercapto group, a mercapto group protected by a protecting group for nucleic acid synthesis, an amino group, a C₁-C₅ alkoxy group, a C₁-C₅ alkylthio group, a C₁-C₆ cyanoalkoxy group, or an amino group substituted with a C₁-C₅ alkyl group]. Non-limiting examples of such BNA comprise methyleneoxy(4'-CH₂-O-2') BNA (LNA, Locked Nucleic Acid (Registered Trademark), also known as 2',4'-BNA) (e.g., α-L-methyleneoxy(4'-CH₂-O-2') BNA or β-D-methyleneoxy(4'-CH₂-O-2') BNA), ethyleneoxy(4'-(CH₂)₂-O-2') BNA (also known as ENA), β-D-thio(4'-CH₂-S-2') BNA, aminooxy(4'-CH₂-O-N(R₃)-₂') BNA, oxyamino(4'-CH₂-N(R₃)-O-2') BNA (also known as 2',4'-BNA^{NC}; R=H is 2',4'-BNA^{NC}[N-H], R=Me is 2',4'-BNA^{NC}[N-Me]), 2',4'-BNA^{coc}, 3'-amino-2',4'-BNA, 5'-methyl BNA, (4'-CH(CH₃)-O-2') BNA (also known as cEt BNA), (4'-CH(CH₂OCH₃)-O-2') BNA (also known as cMOE BNA), amide BNA (amide-bridged nucleic acid) or (4'-C(O)-N(R)-2') BNA (R=H, or Me) (also known as AmNA; R=H is AmNA[N-H], R=Me is AmNA[N-Me]), guanidine BNA (also known as GuNA (e.g., R=H is GuNA[N-H], R=Me is GuNA[N-Me]), amine BNA (also known as 2'-Amino-LNA) (e.g., 3-(Bis(3-aminopropyl)amino)propanoyl substitution product), 2'-O,4'-C-spirocyclopropylene-bridged nucleic acid (also known as scpBNA), and other BNA known to those skilled in the art. Non-limiting examples of such BNA nucleoside comprise methyleneoxy(4'-CH₂-O-2') BNA nucleoside (also known as LNA nucleoside or 2',4'-BNA nucleoside) (e.g., α-L-methyleneoxy(4'-CH₂-O-2') BNA nucleoside, β-D-methyleneoxy(4'-CH₂-O-2') BNA nucleoside), ethyleneoxy(4'-(CH₂)₂-O-2') BNA nucleoside (also known as ENA nucleoside), β-D-thio(4'-CH₂-S-2') BNA nucleoside, aminooxy(4'-CH₂-O-N(R₃)-2') BNA nucleoside, oxyamino(4'-CH₂-N(R₃)-O-2') BNA nucleoside (also known as 2',4'-BNA^{NC} nucleoside; R=H is 2',4'-BNA^{NC}[N-H] nucleoside, and R=Me is 2',4'-BNA^{NC}[N-Me] nucleoside), 2',4'-BNA^{coc} nucleoside, 3'-amino-2',4'-BNA nucleoside, 5'-methyl BNA nucleoside, (4'-CH(CH₃)-O-2') BNA nucleoside (also known as cEt nucleoside), (4'-CH(CH₂OCH₃)-O-2') BNA nucleoside (also known as cMOE nucleoside), amide BNA nucleoside or (4'-C(O)-N(R)-2') BNA nucleoside (R=H, or Me) (also known as AmNA nucleoside; R=H is AmNA[N-H] nucleoside, and R=Me is AmNA[N-Me] nucleoside)), guanidine BNA nucleoside (GuNA nucleoside (e.g., R=H is also known as GuNA[N-H] nucleoside, and R=Me is also known as GuNA[N-Me] nucleoside)), amine BNA nucleoside (also known as 2'-Amino-LNA nucleoside) (e.g., 3-(Bis(3-aminopropyl)amino)propanoyl-substituted nucleoside), 2'-O,4'-C-spirocyclopropylene-bridged nucleoside (also known as scpBNA nucleoside), and other BNA nucleosides known to those skilled in the art.

A "modified internucleoside bond" means herein an internucleoside bond that has a substitution or any change from a naturally occurring internucleoside bond (i.e., phosphodiester linkage). A modified internucleoside bond comprises an internucleoside bond that comprises a phosphorus atom, and an internucleoside bond that does not comprise a phosphorus atom. Typical examples of the phosphorus-containing internucleoside bond comprise, but are not limited to, a phosphodiester linkage, a phosphorothioate linkage, a phosphorodithioate linkage, a phosphotriester linkage, an alkylphosphonate linkage, an alkylthiophosphonate linkage, a methylthiophosphonate linkage, a boranophosphate linkage, an internucleoside bond comprising a cyclic guanidine moiety, and a phosphoramidate linkage. A phosphorothioate linkage refers to an internucleoside bond in which an unbridged oxygen atom in a phosphodiester linkage is substituted with a sulfur atom. A method for preparing a phosphorus-containing a phosphorus-free linkage is well known. It is preferable that a modified internucleoside bond is a linkage having a higher resistance to a nuclease than a naturally occurring internucleoside bond.

The term "nucleobase" or "base" used herein is a base component (heterocyclic moiety) constituting a nucleic acid. As the component, mainly adenine, guanine, cytosine, thymine, and uracil are known. The "nucleobase" or "base" herein encompasses both of a modified or an unmodified nucleobase (base), unless otherwise specified. Accordingly, a purine base may be any of a modified or an unmodified purine base, unless otherwise specified. In addition, a pyrimidine base may be any of a modified or an unmodified pyrimidine base, unless otherwise specified.

A "modified nucleobase" or a "modified base" means any nucleobase other than adenine, cytosine, guanine, thymine, or uracil. The term "unmodified nucleobase" or "unmodified base" (a natural nucleobase) means adenine (A) and guanine (G), which are purine bases, and thymine (T), cytosine (C), and uracil (U), which are pyrimidine bases. Examples of a modified nucleobase comprise, but are not limited to, hypoxanthine, 5-methylcytosine, 5-fluorocytosine, 5-bromocytosine, 5-iodocytosine, and N4-methylcytosine; N6-methyladenine or 8-bromoadenine; 2-thio-thymine; and N2-methylguanine or 8-bromoguanine. The modified nucleobase is preferably 5-methylcytosine.

The term "complementary" as used herein refers to the relationship that nucleobases can form via hydrogen bonds so-called Watson-Crick base pairs (natural base pairs) or non-Watson-Crick base pairs (e.g., Hoogsteen base pairs). In the present invention, the adapter sequence that enables formation of multiplex strands is not necessarily required to be completely complementary, and it is acceptable if the base sequence has a complementarity of at least 70%, preferably at least 80%, and further preferably at least 90% (e.g., 95%, 96%, 97%, 98%, or 99% or more). Base sequence complementarity can be determined by using a BLAST program or the like. Those skilled in the art can easily determine the conditions (temperature, salt concentration, and the like) under which two strands can anneal or hybridize, taking into account the degree of complementarity between the strands.

Hybridization conditions may be variously stringent, e.g., low-stringent and high-stringent conditions. Low-stringent conditions may be relatively low temperature and high salt concentration conditions, e.g., 30°C, 2 × SSC, 0.1% SDS. High-stringent conditions may be relatively high temperature and low salt concentration conditions, e.g., 65°C, 0.1 × SSC, 0.1% SDS. The stringency of hybridization can be adjusted by changing conditions such as temperature and salt concentration. In this connection, 1 × SSC comprises 150 mM sodium chloride and 15 mM sodium citrate.

A "subject" herein refers to the object to which the nucleic acid agent or the pharmaceutical composition of the present invention is applied. A subject comprises an individual as well as an organ, a tissue, and a cell. When the subject is an individual, any animal including a human may be applicable. For example, in addition to a human, a variety of domestic animals, domestic fowls, pets, and laboratory animals are included. Without limitation, the subject may be an individual in need of a decrease in the aggregate of TDP-43 protein or an individual in need of treatment or prevention of central nervous system diseases such as ALS.

The term "plurality" means herein an integer of 2 or more, e.g., an integer of 2 to 10, 2 to 7, 2 to 5, 2 to 4, or 2 to 3.

The "base identity" herein refers to the percentage of matched bases out of the total number of bases in two nucleic acid sequences to be compared, when the sequences are aligned by inserting gaps into one or both sequences as necessary to maximize the number of matched bases.

### 1-3. Configuration

The multivalent binding nucleic acid agent of the present invention (hereinafter simply referred to as "nucleic acid agent of the present invention") comprises two or more nucleic acid strands. In the nucleic acid agent of the present invention, each of two or more nucleic acid strands comprises or consists of a TDP-43 binding sequence capable of binding to TDP-43 protein or a fragment thereof.

The number of the nucleic acid strands in the "two or more nucleic acid strands" herein is not particularly restricted and may be two, three, four, five, six, seven, eight, nine, ten, fifteen, or twenty or more, e.g., two to five, or two or three.

When the strands of two or more nucleic acid strands are distinguished herein, the strands are referred to as a first nucleic acid strand, a second nucleic acid strand, a third nucleic acid strand, and the like. For example, when the nucleic acid agent of the present invention comprises two nucleic acid strands, the first and second nucleic acid strands each comprise a TDP-43 binding sequence. When the multivalent binding nucleic acid agent of the present invention comprises three nucleic acid strands, the first, second, and third nucleic acid strands each comprise a TDP-43 binding sequence. The TDP-43 binding sequences in the strands may be the same or different.

In one embodiment, the multivalent binding nucleic acid agent of the present invention comprises a first nucleic acid strand and a second nucleic acid strand. In this embodiment, the first nucleic acid strand and the second nucleic acid strand are bound via a linker or comprise an adapter sequence, which allows the first nucleic acid strand and the second nucleic acid strand to form a double strand.

In a further embodiment, the multivalent binding nucleic acid agent of the present invention comprises a first nucleic acid strand, a second nucleic acid strand, and a third nucleic acid strand. In this embodiment, the first nucleic acid strand and the second nucleic acid strand, and the second nucleic acid strand and the third nucleic acid strand are connected via a linker. Alternatively, each of the first to third nucleic acid strands comprises an adapter sequence which allows the formation of a double strand between the first and second nucleic acid strands and the second and third nucleic acid strands.

The multivalent binding nucleic acid agent of the present invention may, in addition to two or more nucleic acid strands comprising a TDP-43 binding sequence, further comprise a nucleic acid strand not comprising a TDP-43 binding sequence or may not comprise a nucleic acid strand not comprising a TDP-43 binding sequence.

In the nucleic acid agent of the present invention, each of the two or more nucleic acid strands can comprise a nucleic acid sequence other than the TDP-43 binding sequence in addition to the TDP-43 binding sequence, while, in some embodiments, each of the two or more nucleic acid strands consists of a TDP-43 binding sequence.

Each strand of the two or more nucleic acid strands can comprise natural nucleoside and/or non-natural nucleoside, and, for example, can also be composed of bridged nucleoside in which nucleosides are linked via a natural or modified internucleoside bond and/or modified nucleoside such as 2' modified nucleoside.

The base length of each strand of the two or more nucleic acid strands is not particularly limited and can be independently selected. The base length of each strand may be the same or different, and may be, for example, at least 9 bases in length, 10 bases in length, 11 bases in length, 12 bases in length, 13 bases in length, 14 bases in length, or 15 bases in length, and/or may be 500 bases in length, 200 bases in length, 100 bases in length, 50 bases in length, 40 bases in length, 35 bases in length, 30 bases in length, 25 bases in length, 24 bases in length, 23 bases in length, 22 bases in length, 21 bases in length, 20 bases in length, 19 bases in length, 18 bases in length, 17 bases in length, or 16 bases in length or less. Exemplary ranges include 10 to 100 bases in length, 12 to 60 bases in length, or 15 to 40 bases in length.

The nucleic acid agent of the present invention comprises a spacer which links two or more nucleic acid strands. The "spacer" herein refers to a part, a molecule, and/or a complex, part of which is bound to one or at least two of the two or more nucleic acid strands comprised in the nucleic acid agent of the present invention and which has a sufficient rigidity or length, or a combination thereof which allows the TDP-43 binding sequences of the nucleic acid strands to be positioned at positions spatially separated from each other. Examples of the spacer include a linker described below which directly binds two or more nucleic acid strands and a nucleic acid complex such as a multiplex strand formed by an adapter sequence described below. The length of the spacer is not restricted insofar as it is a length in which the aggregate formation of TDP-43 protein can be suppressed and is, for example, 1 nm or more, preferably 2 nm or more, 3 nm or more, or 4 nm or more. The upper limit of the length of the spacer is not particularly restricted, and may be, for example, 100 nm or less or 50 nm or less, preferably 20 nm or less, and more preferably 10 nm or less or 8 nm or less.

In some embodiments of the nucleic acid agent of the present invention, two or more nucleic acid strands are bound via a linker. To suppress the aggregate formation by two or more TDP-43 proteins (e.g., monomeric TDP-43 protein) bound to the TDP-43 binding sequences, the linker preferably has a rigidity and/or a length which allows them to be positioned at positions spatially separated from each other. A rigid linker is preferable which is capable of maintaining a distance (e.g., restricting or preventing contact) between two or more TDP-43 proteins bound to the TDP-43 binding sequence. Herein, the suppression of the aggregate formation by two or more TDP-43 proteins may be based on a reduced binding between the C-terminal regions of the TDP-43 proteins or an increased phase separation thereof.

The specific configuration of the linker is not particularly restricted, and the linker may be any linker which is not bound to TDP-43 protein. Examples of the linker include linkers comprising nucleic acids, peptides, polyether groups, and/or hydrocarbon groups and linkers composed of (or consisting of) nucleic acids, peptides, polyether groups, and/or hydrocarbon groups (i.e., nucleic acid strands, peptide strands, polyether strands, and/or hydrocarbon strands).

In one embodiment, the nucleic acid comprised in the linkers or constituting the linkers described above consists of one or 2 to 200 (e.g., 2 to 100 or 2 to 50) nucleosides linked via internucleoside bonds. The number of nucleosides linked via internucleoside bonds may be, for example, 3 to 40, 4 to 35, 5 to 30, 6 to 25, or 10 to 20, preferably 12 to 24 or 12 more and less than 24, e.g., 11, 12, 13, 14, or 15. The nucleoside described above can comprise natural nucleoside and/or non-natural nucleoside. The type of the nucleic acid is not particularly restricted, and may be, for example, peptide nucleic acid.

In one embodiment, the length of the peptide comprised in the linker or constituting the linker described above is not limited, and examples thereof include 3 to 100 amino acids in length, 5 to 50 amino acids in length, and 10 to 40 amino acids in length, and is preferably 15 to 30 amino acids in length or 20 to 25 amino acids in length. The type of an amino acid residue constituting the peptide can be selected as appropriate considering a rigidity and a three-dimensional structure required for the linker.

In one embodiment, the polyether group comprised in the linker or constituting the linker described above is not particularly restricted insofar as it is a linear or branched polymer having an ether bond in the main chain and is preferably a linear or branched polymer comprising alkylene glycol units or polyalkylene glycol units. The number of molecules of the alkylene glycol units or the polyalkylene glycol units constituting the main chain of the linker is not limited, and may be, for example, 1 to 20, 2 to 16, or 3 to 12. The alkylene glycol units or the polyalkylene glycol units are preferably ethylene glycol units or polyethylene glycol (PEG) units. The ethylene glycol or the polyethylene glycol may be, for example, ethylene glycol, diethylene glycol, triethylene glycol, tetraethylene glycol, pentaethylene glycol, hexaethylene glycol, heptaethylene glycol, octaethylene glycol, nonaethylene glycol, decaethylene glycol, and the like, and is preferably ethylene glycol, diethylene glycol, triethylene glycol, or tetraethylene glycol.

In one embodiment, the hydrocarbon groups comprised in the linker or constituting the linker described above are optionally substituted C₂-C₃₀ hydrocarbon groups. The number of carbons constituting the hydrocarbon groups may be 3 to 25, 4 to 20, 5 to 18, or 10 to 15, and, for example, may be 11 to 13 or 12 and is preferably 3 to 6. The hydrocarbon groups may be saturated alkylene groups or unsaturated alkenylene groups or alkynylene groups. The alkenylene groups or the alkynylene groups are preferable because they can enhance the rigidity of the linker. The hydrocarbon groups may be either linear or branched. The substituent may be a hydroxyl group, an amino group, an alkoxy group, a carboxy group, a benzyl group, a phenyl group, a nitro group, a thiol group, a thioalkoxy group, a halogen group, an alkyl group, an aryl group, an alkenyl group, and/or an alkynyl group.

The linker may be any of a cleavable linker and an uncleavable linker. A "cleavable linker" refers to a linker that can be cleaved under physiological conditions, e.g., in a cell or in an animal body (e.g., in a human body). A cleavable linker is selectively cleaved by an endogenous enzyme such as a nuclease. Examples of a cleavable linker include, but are not limited to, an amide, an ester, one or both esters of a phosphodiester, a phosphoester, a carbamate, and a disulfide bond, as well as a natural DNA linker. An "uncleavable linker" refers to a linker that is not cleaved under physiological conditions, e.g., in a cell or in an animal body (e.g., in a human body). Examples of an uncleavable linker include, but are not limited to, a phosphorothioate linkage, modified or unmodified deoxyribonucleosides linked via a phosphorothioate linkage, a linker consisting of modified or unmodified ribonucleosides, or the like. The linker is more preferably the uncleavable linker.

Further specific examples of the linker can be selected as appropriate from known linkers usable in linking nucleic acids in the art. Further, a linker synthesis method and a method for binding nucleic acid strands via a linker are also known in the art.

In one embodiment, the linker comprises a region consisting of a single-stranded nucleic acid. The region consisting of a single-stranded nucleic acid can comprise any secondary structure and can comprise a hairpin (loop) structure, a stem (loop) structure, and/or a pseudoknot structure. The hairpin structure may be, for example, a guanine quadruplex (G-quadruplex; G4) structure or an adenine quadruplex (A-quadruplex; A4) structure. These quadruplex structures may be any of a parallel type, an antiparallel type, or a hybrid type. The region consisting of a single-stranded nucleic acid can comprise natural nucleoside and/or non-natural nucleoside. For example, the region consisting of a single-stranded nucleic acid can be composed of bridged nucleoside in which nucleosides are linked via a natural or modified internucleoside bond and/or modified nucleoside such as 2' modified nucleoside.

In a further embodiment, the multivalent binding nucleic acid agent of the present invention can further comprise a complementary strand comprising a complementary base sequence at least in part of the region consisting of a single-stranded nucleic acid described above. The complementary strand can improve the rigidity of the linker by forming a multiplex strand structure (e.g., a duplex strand structure, a triplex strand structure, or a quadruplex or higher strand structure) with the region consisting of a single-stranded nucleic acid described above. The region consisting of a single-stranded nucleic acid and the complementary strand can comprise any secondary structure, and can comprise, for example, a stem structure, a bulge (loop) structure, an internal loop structure, a branched loop structure, or the like. The complementary strand can also comprise natural nucleoside and/or non-natural nucleoside, and, for example, can be composed of bridged nucleoside in which nucleosides are linked via a natural or modified internucleoside bond and/or 2' modified nucleoside.

In a further embodiment, the region consisting of a single-stranded nucleic acid comprises a non-complementary base (mismatched base), and/or one or more insertion sequences and/or deletions, relative to the complementary strand. For example, the number of the non-complementary bases may be 1 to 3, the insertion sequence may consist of 1 to 8 bases, and the deletion may consist of one to four consecutive bases. The introduction of the non-complementary base, the insertion sequence, and/or the deletion also enables the formation of the secondary structure as described above.

The base lengths of the region consisting of a single-stranded nucleic acid and the complementary strand described above are not particularly limited, and may be at least 6 bases in length, 7 bases in length, 8 bases in length, 9 bases in length, 10 bases in length, 11 bases in length, 12 bases in length, 13 bases in length, 14 bases in length, or 15 bases in length, and/or may be 50 bases in length, 40 bases in length, 35 bases in length, 30 bases in length, 25 bases in length, 24 bases in length, 23 bases in length, 22 bases in length, 21 bases in length, 20 bases in length, 19 bases in length, 18 bases in length, 17 bases in length, or 16 bases in length or less. Exemplary ranges include 8 to 50 bases in length, 12 to 40 bases in length, or 15 to 30 bases in length, and the base length is preferably 12 to 24 bases in length or 12 bases or more in length and less than 24 bases in length.

In some embodiments of the nucleic acid agent of the present invention, two or more nucleic acid strands comprise an adapter sequence which enables the formation of a multiplex strand between two or more nucleic acid strands.

The "adapter sequence" means herein two or more base sequences that enable formation of multiplex strands by hybridization or nucleic acids consisting of the sequences, and can be sequences having complementarity in at least part thereof. For example, when two nucleic acid strands form a duplex strand between nucleic acid strands, a first nucleic acid strand comprises, in addition to the TDP-43 binding sequence described above, a first adapter sequence that does not bind to TDP-43 and a second nucleic acid strand comprises, in addition to the TDP-43 binding sequence, a second adapter sequence comprising a complementary base sequence in at least part of the first adapter sequence, and thus the two nucleic acid strands can form a duplex strand between the adapter sequences. In two or more nucleic acid strands, the adapter sequence may be positioned either on the 5' side and/or the 3' side of the TDP-43 binding sequence. For example, the first and second adapter sequences are positioned on the 5' side of the TDP-43 binding sequence in the first and second nucleic acid strands, respectively, or the first and second adapter sequences are positioned on the 3' side of the TDP-43 binding sequence in the first and second nucleic acid strands, respectively. This is preferable because the first and second nucleic acid strands constituted as described above allows the TDP-43 binding sequence of each nucleic acid strand to be positioned at positions spatially separated from each other.

The adapter sequence described above can comprise any secondary structure, and can comprise, for example, a stem structure, a bulge (loop) structure, an internal loop structure, a branched loop structure, or the like. The adapter sequence can also comprise natural nucleoside and/or non-natural nucleoside, and can also be composed of, for example, bridged nucleoside in which nucleosides are linked via a natural or modified internucleoside bond and/or 2' modified nucleoside.

In a further embodiment, the adapter sequence comprises a non-complementary base (mismatched base), and/or one or more insertion sequences and/or deletions of one or more bases. For example, the number of the non-complementary bases may be 1 to 3, the insertion sequence may consist of 1 to 8 bases, and the deletion may consist of one to four consecutive bases. The introduction of the non-complementary base, the insertion sequence, and/or the deletions also enables the formation of the secondary structure described above.

The base length of the adapter sequence is not particularly limited, and may be at least 6 bases in length, 7 bases in length, 8 bases in length, 9 bases in length, 10 bases in length, 11 bases in length, 12 bases in length, 13 bases in length, 14 bases in length, or 15 bases in length, and/or may be no more than 50 bases in length, 40 bases in length, 35 bases in length, 30 bases in length, 25 bases in length, 24 bases in length, 23 bases in length, 22 bases in length, 21 bases in length, 20 bases in length, 19 bases in length, 18 bases in length, 17 bases in length, or 16 bases in length or less. Exemplary ranges include 8 to 50 bases in length, 12 to 40 bases in length, or 15 to 30 bases in length, and the base length is preferably 8 to 30 bases in length or 10 to 2 bases in length.

In one embodiment of the multivalent binding nucleic acid agent of the present invention, a functional moiety may be bound to at least one of two or more nucleic acid strands. The bond between the nucleic acid strand and the functional moiety may be a direct bond or an indirect bond via another substance. It is preferable that the nucleic acid strand and the functional moiety are directly bound via a covalent bond, an ionic bond, a hydrogen bond, or the like, and, from the viewpoint of obtaining a more stable bond, a covalent bond is more preferable.

In one embodiment, the structure of the "functional moiety" is not particularly restricted and provides a desired function to a divalent binding nucleic acid which binds the functional moiety. Examples of the desired function include a labeling function, a purification function, a delivery function to a target, and the like. Examples of a moiety that provides a labeling function include compounds such as a fluorescent protein and luciferase. Examples of a moiety that provides a purifying function include compounds such as biotin, avidin, His-tag peptide, GST-tag peptide, and FLAG-tag peptide. From the viewpoint that the nucleic acid agent is efficiently delivered to a target site at a high specificity and TDP-43 protein is effectively controlled by the nucleic acid agent, a molecule having delivery activity to a target site can be bound to at least one of two or more nucleic acid strands as the functional moiety. Examples of a moiety that provides a delivery function to a target include lipid, antibody, aptamer, a ligand to a particular receptor, and the like.

In one embodiment, at least one of the two or more nucleic acid strands (e.g., first and/or second nucleic acid strand(s)) is bound to a lipid. Lipids include, but are not limited to, tocopherol, cholesterol, fatty acids, phospholipids, and their analogues; folic acid, vitamin C, vitamin B1, vitamin B2; estradiol, androstane, and their analogues; and steroids and their analogues. The lipid may be tocopherol or an analogue thereof and/or cholesterol or an analogue thereof, a substituted or unsubstituted C₁-C₃₀ alkyl group, a substituted or unsubstituted C₂-C₃₀ alkenyl group, or a substituted or unsubstituted C₁-C₃₀ alkoxy group.

The functional moiety may be linked to the 5' end, the 3' end, or both ends of at least one of the two or more nucleic acid strands (e.g., the first and/or second nucleic acid strand(s)). Alternatively, the functional moiety may be linked to nucleotide inside at least one of the two or more nucleic acid strand (e.g., the first and/or second nucleic acid strand(s)).

The functional moiety may also be bound to at least one of the two or more nucleic acid strands (e.g., the first and/or second nucleic acid strand(s)) via a cleavable or uncleavable linker.

In some embodiments, theTDP-43 binding sequences in the two or more of nucleic acid strands of the multivalent binding nucleic acid agent of the present invention are bound to the RNA recognition motif (RRM) of TDP-43 protein. TDP-43 protein has two RRMs of RRM1 and RRM2, and the TDP-43 binding sequence may be bound to either or both of them.

In one embodiment, the TDP-43 binding sequence which binds to the RNA recognition motif of TDP-43 protein consists of or comprises a repeat sequence represented by the following formula (I):

(X₀ G X₁ X₂ X₃ ... Xₘ)ₙ (I)

wherein, X₀ is T or U; X₁, X₂, X₃, ..., and Xₘ are independently any of A, C, G, T, or U, and may be identical or different, or X₁, X₂, X₃, ..., and Xₘ are absent; m is 1 to 10; and n represents a repeat number and is 2 to 50, preferably 5 to 25 or 4 to 15.

Specific examples of the repeat sequence represented by Formula (I) above include a repeat sequence of the sequence UGGAA represented by the following formula (II):

(UGGAA)ₙ (II)

wherein, n represents a repeat number and is 2 to 50 and preferably 15 to 25.

In a further embodiment, the TDP-43 binding sequence which binds to the RNA recognition motif of TDP-43 protein consists of or comprises a repeat sequence represented by the following formula (III):

(U_{Me} G X₁ X₂ X₃ ... Xₘ)ₙ (III)

wherein, U_{Me} is 2'-O-methyl-modified nucleoside, G may be natural ribonucleoside or 2'-modified nucleoside such as 2'-O-metyl-modified nucleoside; X₁, X₂, X₃, ... Xₘ are independently any of A, C, G, T, or U, and may be identical or different, or X₁, X₂, X₃, ... Xₘ are absent, and X₁, X₂, X₃, ... Xₘ may be natural ribonucleoside or 2'-modified nucleoside such as 2'-O-metyl-modified nucleoside; m is 1 to 10, and n represents a repeat number and is 2 to 30 and preferably 5 to 25 or 4 to 15.

Examples of the TDP-43 binding sequence comprising any of Formulae (I) to (III) above include AUG12 consisting of the base sequence (GUGUGAAUGAAU) shown in SEQ ID NO: 6 and CLIP_34 consisting of the base sequence (GAGAGAGCGCGUGCAGAGACUUGGUGGUGCAUAA) shown in SEQ ID NO: 7 (Lukavsky, P.J. et al., Nat Struct MolBiol, 2013, 20(12):1443-9.; Grese Z. R. et al., EMBO Rep, 2021, 22(12):e53632.; Ayala Y. M. et al., EMBO J, 2011, 30(2):277-88.; Mann, J. R. et al., Neuron, 2019, 102(2):321-338. e8.).

Non-limiting specific examples of the TDP-43 binding sequence which binds to the RNA recognition motif of TDP-43 protein are shown below: (U_{Me}G)₅₋₁₅, (U_{Me}G)₁₂, (U_{Me}G_{Me})₅₋₁₅, (U_{Me}G_{Me})₁₂, (U_{Me}GGAA)₅₋₁₀, (U_{Me}GGAA)₈, (U_{Me}G_{Me}GAA)₅₋₁₀, (U_{Me}G_{Me}GAA)₈, (U_{Me}G_{Me}G_{Me}AA)₅₋₁₀, (U_{Me}G_{Me}G_{Me}AA)₈, (U_{Me}G_{Me}G_{Me}A_{Me}A)₅₋₁₀, (U_{Me}G_{Me}G_{Me}A_{Me}A)₈, (U_{Me}G_{Me}G_{Me}A_{Me}A_{Me})₅₋₁₀, (U_{Me}G_{Me}G_{Me}A_{Me}A_{Me})₈, (U_{Me}GG_{Me}AA)₅₋₁₀, (U_{Me}GG_{Me}AA)₈, (U_{Me}GGA_{Me}A)₅₋₁₀, (U_{Me}GGA_{Me}A)₈, (U_{Me}GGAA_{Me})₅₋₁₀, (U_{Me}GGAAA_{Me})₈, (U_{Me}G_{Me}GA_{Me}A)₅₋₁₀, (U_{Me}G_{Me}GA_{Me}A)₈, (U_{Me}G_{Me}GAA_{Me})₅₋₁₀, (U_{Me}G_{Me}GAA_{Me})₈, (U_{Me}GG_{Me}A_{Me}A)₅₋₁₀, (U_{Me}GG_{Me}A_{Me}A)₈, (U_{Me}GG_{Me}AA_{Me})₅₋₁₀, (U_{Me}GG_{Me}AA_{Me})₈, (U_{Me}GGA_{Me}A_{Me})₅₋₁₀, (U_{Me}GGA_{Me}A_{Me})₈, (U_{Me}GG_{Me}A_{Me}A_{Me})₅₋₁₀, (U_{Me}GG_{Me}A_{Me}A_{Me})₈, (U_{Me}G_{Me}GA_{Me}A_{Me})₅₋₁₀, (U_{Me}G_{Me}GA_{Me}A_{Me})₈, (U_{Me}G_{Me}G_{Me}AA_{Me})₅₋₁₀, (U_{Me}G_{Me}G_{Me}AA_{Me})₈. In the above-described specific examples, U_{Me}, A_{Me}, and G_{Me} represent the 2'-O-methyl of modified nucleoside, and A and G represent unmodified ribonucleoside. In the above-described specific examples, the 2'-O-methyl modified nucleoside can be independently substituted by 2'-O-methoxyethyl-modified nucleoside or 2'-O-[2-(N-methylcarbamoyl)ethyl]-modified nucleoside, and U can be independently substituted by T.

In a further embodiment, the TDP-43 binding sequence which binds to the RNA recognition motif of TDP-43 protein consists of or comprises a repeat sequence represented by the following formula (IV):

(XG)ₙ (IV)

wherein, X represents T or U.

In Formula (IV), n representing a repeat number is 3 or more, and the repeat sequence of (IV) above in this case is 6 mer or more, which is considered to be a sufficient length which allows the TDP-43 binding sequence to bind to fragmented TDP-43 protein. The upper limit of n is not restricted, and the repeat sequence of Formula (IV) above may be a length which allows the TDP-43 binding sequence to bind to one (1 molecule) or two (2 molecules) or more TDP-43 proteins or a fragment thereof. For example, n may be 3 to 30, 3 to 20, or 3 to 10.

In a preferable embodiment, the TDP-43 binding sequence has a length which allows the TDP-43 binding sequence to bind to one TDP-43 protein or a fragment thereof. In this case, in Formula (IV), n may be, for example, 6 or less or 5 or less, with n ranging from 3 to 6 or 3 to 5. In Formula (IV) above in this case, the repeat sequence is 6 mer to 12 mer or 6 mer to 10 mer. The n instances of X comprised in Formula (IV) above may each independently be T or U, but all may be T or all may be U. Thus, in a further embodiment, the TDP-43 binding sequence consists of or comprises a repeat sequence represented by the following formula (V):

(TG)ₙ (V).

In a still further embodiment, the TDP-43 binding sequence consists of or comprises a repeat sequence represented by the following formula (VI):

(UG)ₙ (VI).

The TDP-43 binding sequence or the repeat sequence shown in Formulae (I) to (VI) above can comprise natural nucleoside and/or non-natural nucleoside. The bond between nucleosides in the TDP-43 binding sequence or the repeat sequence shown in Formulae (I) to (VI) above may be a natural type or modified internucleoside bond or a combination thereof.

In one embodiment, the repeat sequence represented by Formula (I), (II), (IV), (V), or (VI) above is composed of RNA nucleosides. The repeat sequence in this case is the repeat RNA sequence.

For example, the TDP-43 binding sequence can be composed of bridged nucleosides and/or 2' modified nucleosides linked via a natural or modified internucleoside bond. In one embodiment, non-natural ribonucleoside is 2'-O-methyl-modified nucleoside, 2'-O-methoxyethyl-modified nucleoside, or 2'-O-[2-(N-methylcarbamoyl)ethyl]-modified nucleoside.

In one embodiment, at least the first U or T in the repeat sequence shown in Formula (I), (II), (IV), (V), or (VI) above is 2' modified nucleoside or 2' modified ribonucleoside. In this embodiment, the other bases of the repeat sequence may or may not be 2' modified nucleoside or 2' modified ribonucleoside, but the second G is preferably 2' modified ribonucleoside. The above-described modified ribonucleoside may be, but is not restricted to, 2'-O-methyl-modified nucleoside, 2'-O-methoxyethyl-modified nucleoside, 2'-O-[2-(N-methylcarbamoyl)ethyl]-modified nucleoside, or a combination thereof.

In a further embodiment, all bases of the repeat sequence shown in Formulae (I) to (VI) above may be 2' modified ribonucleoside. The above-described modified ribonucleoside may be, but is not restricted to, 2'-O-methyl-modified nucleoside, 2'-O-methoxyethyl-modified nucleoside, 2'-O-[2-(N-methylcarbamoyl)ethyl]-modified nucleoside, or a combination thereof.

### 1-4. Method for producing multivalent binding nucleic acid agent

Those skilled in the art can produce the multivalent binding nucleic acid agent of the present invention by appropriately selecting a publicly known method. Usually, without limitation, firstly each of two or more nucleic acid strands constituting a multivalent binding nucleic acid agent is designed and produced. Then, based on the information on the designed base sequences, each nucleic acid strand may be synthesized using a commercially available automatic nucleic acid synthesizer such as that from GE Healthcare, Thermo Fisher Scientific, or Beckman Coulter. Thereafter, the obtained oligonucleotides can also be purified using a reverse-phase column or the like. A method for linking a spacer such as a linker to a nucleic acid strand is well known in the art. Furthermore, a nucleic acid strand can be obtained by ordering from various manufacturers (e.g., GeneDesign Inc.) by specifying the base sequence and the modification site and type.

### 1-5. Effects

The nucleic acid agent of the present invention can efficiently suppress the aggregate formation by positioning a plurality of TDP-43 proteins or fragments thereof bound to the TDP-43 binding sequence at positions spatially separated from each other, suppressing the approach, contact, or binding between C-terminal regions of a plurality of TDP-43 proteins or fragments thereof bound to the TDP-43 binding sequence, or promoting the phase separation thereof based on the spacer such as the linker positioned between two or more TDP-43 binding sequences. The nucleic acid agent of the present invention can also suppress the aggregate formation of monomers by stabilizing a TDP-43 protein dimer and reducing the ratio of monomers. Surprisingly, the nucleic acid agent of the present invention also exhibits the aggregation suppression effect against mutant TDP-43 protein which cannot form a dimer.

The mechanism of action of the nucleic acid agent of the present invention is not limited, and can be described as follows, for example. Each of two or more TDP-43 binding sequences constituting the nucleic acid agent of the present invention bind to monomeric TDP-43 protein or a fragment thereof. Then, monomers bound to the TDP-43 binding sequences form a multimer such as a dimer by binding between the N-terminal domains.

The present invention also provides a method for treating and/or preventing diseases such as neurodegenerative diseases comprising administering any of the above-described multivalent binding nucleic acid agents to a subject such as a human, and a method for suppressing (in vitro or in vivo) the toxicity, accumulation, and/or aggregation of TDP-43 protein or a fragment thereof (e.g., C-terminal fragment).

The present invention also provides any of the above-described multivalent binding nucleic acid agents for use in treatment and/or prevention of diseases such as neurodegenerative diseases in a subject such as a human.

The present invention also provides any of the above-described multivalent binding nucleic acid agents in the production of a medicine for treating and/or preventing diseases such as neurodegenerative diseases.

The present invention also provides use of any of the above-described multivalent binding nucleic acid agents for suppressing the toxicity, accumulation, and/or aggregation of TDP-43 protein or a fragment thereof (e.g., C-terminal fragment) or for stabilizing a TDP-43 protein dimer.

### 2. Pharmaceutical composition

### 2-1. Overview

A second aspect of the present invention is a pharmaceutical composition. The pharmaceutical composition of the present invention comprises any of the multivalent binding nucleic acid agents described in the aforementioned first aspect as an active ingredient. A pharmaceutical composition of the present invention can exhibit preventive or therapeutic effects against neurodegenerative diseases such as TDP-43 proteinopathy.

Each component that a pharmaceutical composition of the present invention can comprise will be described in detail below.

### 2-2. Configuration

### 2-2-1. Active ingredient

The pharmaceutical composition of the present invention comprises as an active ingredient at least a multivalent binding nucleic acid agent described in the first aspect. A pharmaceutical composition of the present invention may comprise one or two or more kinds of multivalent binding nucleic acid agents.

The amount (content) of the multivalent binding nucleic acid agent in a pharmaceutical composition varies depending on the kind of the multivalent binding nucleic acid agent of the present invention, the delivery site, the dosage form of the pharmaceutical composition, the dose of the pharmaceutical composition, and the kind of a carrier described below. Therefore, it may be determined as appropriate by taking the respective conditions into consideration. Usually, it may be prepared so that an effective amount of the multivalent binding nucleic acid agent is comprised in a single dose of the pharmaceutical composition. An "effective amount" refers to an amount that is necessary for the multivalent binding nucleic acid agent to function as an active ingredient, and to an amount that has little or no adverse side effect on a living body to which the amount is applied. This effective amount can vary depending on various conditions such as information on the subject, the administration route, and the number of administrations. Ultimately, it is determined by the judgment of a physician, veterinarian, pharmacist, or the like. The "Information on the subject" is various information on an individual of the living body to which the pharmaceutical composition is applied. For example, when the subject is a human, it comprises age, body weight, gender, dietary habit, health status, stage of progression or grade of severity of the disease, drug sensitivity, and presence of a combined drug.

### 2-2-2. Carrier

The pharmaceutical composition of the present invention may comprise a pharmaceutically acceptable carrier. A "pharmaceutically acceptable carrier" refers to an additive commonly used in the field of pharmaceutical preparation. Examples thereof include a solvent, a vegetable oil, a base, an emulsifier, a suspending agent, a surfactant, a pH adjuster, a stabilizer, an excipient, a vehicle, a preservative, a binder, a diluent, an isotonizing agent, a sedative, a bulking agent, a disintegrating agent, a buffering agent, a coating agent, a lubricant, a thickener, a dissolution aid, and other additives.

The solvent may be any of, e.g., water or other pharmaceutically acceptable aqueous solution, and a pharmaceutically acceptable organic solvent. Examples of an aqueous solution include a physiological saline, an isotonic solution comprising glucose or another additive, a phosphate-buffered saline, and a sodium acetate buffer solution. Examples of the additive include D-sorbitol, D-mannose, D-mannitol, sodium chloride, and further a nonionic surfactant at a low concentration, and polyoxyethylene sorbitan fatty acid ester.

The above carrier is used to avoid or decrease degradation of the multivalent binding nucleic acid agent, which is an active ingredient, in vivo by an enzyme and the like, and additionally to facilitate formulation or administration, and to maintain the dosage form and drug efficacy. Therefore, it may be used as appropriate and as needed.

### 2-2-3. Dosage form

There is no particular limitation on the dosage form of the pharmaceutical composition of the present invention as long as the multivalent binding nucleic acid agent described in the first aspect, which is an active ingredient, is delivered to a target site without being inactivated by degradation or the like, and the pharmacological effect of the active ingredient can be produced in vivo.

The specific dosage form varies depending on the administration method and/or medication conditions. The administration methods can be broadly classified into parenteral administration and oral administration, and the dosage form appropriate for the respective administration methods can be selected.

When the administration method is parenteral administration, the preferred dosage form is liquid formulation which can be administered directly to the target site, or administered systemically via the circulatory system. Examples of the liquid formulation include an injectable. An injectable can be formulated by mixing in an appropriate combination with the aforementioned excipient, elixir, emulsifier, suspending agent, surfactant, stabilizer, pH adjuster, etc. in the form of a unit dose required according to the generally approved pharmaceutical practices. In addition, it may be ointment, plaster, cataplasm, transdermal patch, lotion, inhalant, aerosol, eye drop, and suppository.

When the administration method is oral administration, the preferred dosage form includes a solid preparation (comprising a tablet, a capsule, a drop, and a lozenge), granule, dusting powder, powder, and a liquid preparation (comprising an oral liquid preparation, an emulsion, and a syrup). In the case of a solid preparation, if necessary, it may take a dosage form with a coating as publicly known in the art, such as a sugar-coated tablet, a gelatin-coated tablet, an enteric-coated tablet, a film-coated tablets, a double layer tablet, and a multilayer tablet.

There is no particular limitation on the specific shape and size of each of the above-mentioned dosage forms, as long as the respective dosage forms are within the ranges of dosage forms publicly known in the art. As for the manufacturing method of the pharmaceutical composition of the present invention, it may be formulated according to the common procedure in the art.

### 2-3. Dosing form and dose

Herein there is no particular limitation on the preferable dosing form of a pharmaceutical composition. Administration may be systemic or topical. For example, the administration route may be oral or parenteral administration. Specific examples of the parenteral administration include intravenous administration, intraarterial administration, administration by blood transfusion, intraperitoneal administration, intraventricular administration, intrathecal administration, intraocular administration, intramuscular administration, subcutaneous administration (including implantable continuous subcutaneous administration), intradermal administration, intravesical administration, intravaginal administration, rectal administration, inhalation or nasal administration, and tracheal/bronchial administration. When the application target site of the present invention is the brain, intraventricular administration or intrathecal administration, which are the target sites, is preferable. For example, the administration may be intravenous administration, intratumoral administration, hepatic artery infusion, splenic subcapsular infusion, subcutaneous administration, intraventricular administration, or intrathecal administration. The intrathecal administration is, for example, intraventricular administration, posterior fossa puncture, or lumbar puncture. In the intrathecal administration, the pharmaceutical composition of the present invention may be administered using a shunt, indwelling catheter, or subcutaneous port.

When a pharmaceutical composition is applied by administration or ingestion, the administered amount or ingested amount may be, e.g., from 0.00001 mg/kg/day to 10000 mg/kg/day, or from 0.001 mg/kg/day to 100 mg/kg/day for the multivalent binding nucleic acid agent comprised in the pharmaceutical composition. A pharmaceutical composition may be applied by single-dose administration or multiple dose administration. In the case of multiple dose administration, it may be administered daily or at appropriate time intervals (e.g., at intervals of one day, two days, three days, one week, two weeks, or one month), e.g., for 2 to 20 times. A single dose of the above-described multivalent binding nucleic acid agent may be, e.g., 0.001 mg/kg or more, 0.005 mg/kg or more, 0.01 mg/kg or more, 0.25 mg/kg or more, 0.5 mg/kg or more, 1.0 mg/kg or more, 2.0 mg/kg or more, 3.0 mg/kg or more, 4.0 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more. For example, any amount in the range of from 0.001 mg/kg to 500 mg/kg (e.g., 0.001 mg/kg, 0.01 mg/kg, 0.1 mg/kg, 1 mg/kg, 5 mg/kg, 10 mg/kg, 50 mg/kg, 100 mg/kg, or 200 mg/kg) may be selected as appropriate.

For example, when a pharmaceutical composition of the present invention is intrathecally administered, the multivalent binding nucleic acid agent may be administered to a monkey or a human in an amount of 0.01 mg or more, 0.1 mg or more, or 1 mg or more, e.g., 2 mg or more, 3 mg or more, 4 mg or more, 5 mg or more, 10 mg or more, 20 mg or more, 30 mg or more, 40 mg or more, 50 mg or more, 75 mg or more, 100 mg or more, 200 mg or more, 300 mg or more, 400 mg or more, or 500 mg or more, or may be administered in an amount of 0.01 mg to 1000 mg, 0.1 mg to 200 mg, or 1 mg to 20 mg. The pharmaceutical composition may be administered to a mouse in an amount of 1 µg or more.

The multivalent binding nucleic acid agent of the present invention may be administered twice a week for a total of four times at a dose of from 0.01 to 10 mg/kg (e.g., about 6.25 mg/kg). Alternatively, the multivalent binding nucleic acid agent may be administered once or twice a week for total two to four times, e.g., at a frequency of twice a week for total two times, at a dose of from 0.05 to 30 mg/kg (e.g., about 25 mg/kg). By adopting such a dosing regimen (divided administration), the toxicity can be lowered (e.g., avoidance of platelet reduction) compared to a single-dose administration at a higher dose, and the stress to the subject can be reduced.

Even when the pharmaceutical composition is repeatedly administered, its inhibitory effect can be produced additively in a cell. In the case of repeated administration, the efficacy can be improved with certain administration intervals (e.g., half a day or longer).

### 2-4. Disease as subject of application

Diseases to which the pharmaceutical composition is applicable are mainly neurodegenerative diseases. The neurodegenerative diseases refer to progressive diseases in which nerve cells in the central nervous system gradually degenerate due to the accumulation of an abnormal protein aggregate, leading to dysfunction and cell death. Specific examples of neurodegenerative diseases associated with TDP-43 protein include amyotrophic lateral sclerosis which is sporadic amyotrophic lateral sclerosis (ALS) or familial amyotrophic lateral sclerosis, frontotemporal lobar degeneration (FTLD), Alzheimer's disease, dementia with Lewy bodies, Huntington's disease, Parkinson's disease, argyrophilic grain dementia (Grene's disease), Perry syndrome, progressive supranuclear palsy, corticobasal degeneration, multiple system atrophy, and the like. The frontotemporal lobar degeneration may be, for example, Pick's disease. The pharmaceutical composition of the present invention is useful for the prevention or treatment of amyotrophic lateral sclerosis and frontotemporal lobar degeneration among the above. The pharmaceutical composition of the present invention can also be used to prevent or treat neurodegenerative diseases with dementia. A particularly preferable neurodegenerative diseases is TDP-43 proteinopathy, which is caused by TDP-43 protein or a fragment thereof abnormally aggregating and accumulating in the cytoplasm to form intracellular inclusion bodies.

The pharmaceutical composition can be used for animals including a human as a subject. However, there is no specific limitation on animals other than a human, and various domestic animals, domestic fowls, pets, laboratory animals, and the like can be a subject of some embodiments. The subject may be a subject in need of prevention or treatment of neurodegenerative diseases in the central nervous system and/or peripheral nervous system.

### 2-5. Effects

The pharmaceutical composition of the present invention can be used for the prevention or treatment of neurodegenerative diseases. In addition, the pharmaceutical composition of the present invention can suppress the progression of neurodegenerative diseases and prevent recurrence. Further, the pharmaceutical composition of the present invention can be used to suppress or inhibit abnormal intracellular accumulation or cytoplasmic aggregation of TDP-43 protein or a fragment thereof.

The present invention also provides a method for treating and/or preventing neurodegenerative diseases and the like comprising administering the above-described pharmaceutical composition to a subject, e.g., a human.

### Examples

The present invention is described below in more detail by means of Examples. However, the technical scope of the present invention is not limited to these Examples.

### <Example 1: Production of divalent bait nucleic acid>

### (Purpose)

A novel bait nucleic acid is developed in which two TDP-43 binding sequences are positioned on both ends of a spacer, and the aggregate formation between TDP-43 proteins bound to the TDP-43 binding sequences can be suppressed by the presence of the aforementioned spacer. In Examples below, bait nucleic acid which can bind to at least two TDP-43 molecules is referred to as "divalent bait nucleic acid".

### (Method and Results)

Divalent bait nucleic acid capable of binding TDP-43 proteins to positions separated from each other by binding the TDP-43 protein binding sequence, to which one molecule of TDP-43 protein can bind, to both ends of a spacer was produced using a spacer composed of nucleic acids.

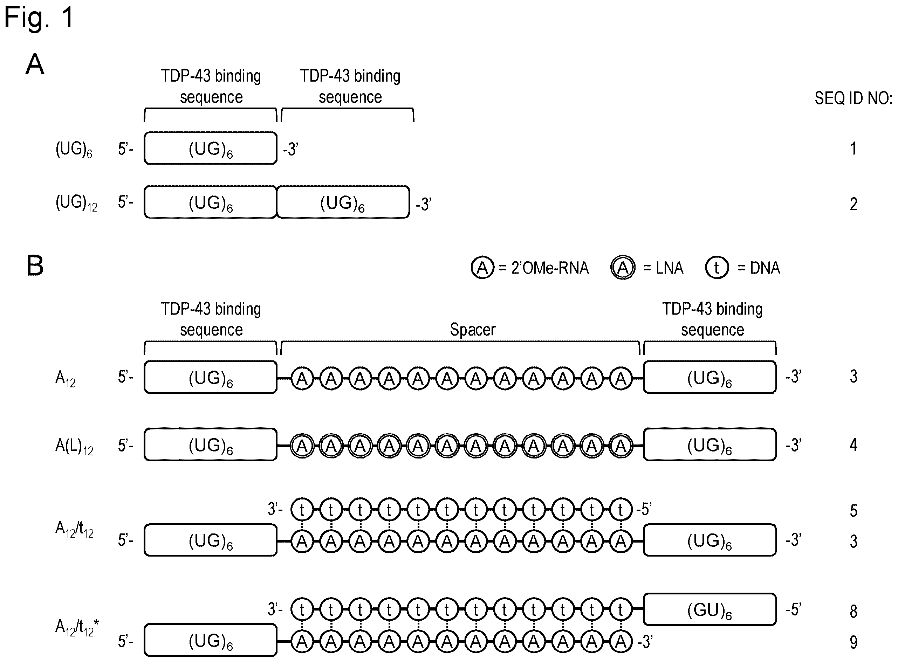

The structures of monovalent bait nucleic acid and divalent bait nucleic acid comprising no spacer used as controls and divalent bait nucleic acids comprising nucleic acids as a spacer for binding the TDP-43 binding sequence to both ends are shown in Table 1 below and Figure 1.

**[Table 1]**

| Table 1: Structure of bait nucleic acid | | | |
|---|---|---|---|
| Name of bait nucleic acid | | Sequence (5'-3') | SEQ ID NO |
| (UG)₆ | | UGUGUGUGUGUG | 1 |
| (UG)₁₂ | | UGUGUGUGUGUG UGUGUGUGUGUG | 2 |
| A₁₂ | | UGUGUGUGUGUG AAAAAAAAAAAA UGUGUGUGUGUG | 3 |
| A(L)₁₂ | | UGUGUGUGUGUG AAAAAAAAAAAA UGUGUGUGUGUG | 4 |
| A₁₂/t₁₂ | A₁₂ strand | UGUGUGUGUGUG AAAAAAAAAAAA UGUGUGUGUGUG | 3 |
| | t₁₂ strand | tttttttttttt | 5 |

| | | | |
|---|---|---|---|
| Upper case letter: 2'-O-methyl-RNA; Underlined upper case letter: LNA; Lower-case letter: DNA; Internucleoside linkages are all phosphodiester linkages. | | | |

Bait nucleic acid (UG)₆ is monovalent bait nucleic acid which is composed of 2'-O-methyl-RNA nucleosides and to which one TDP-43 protein can bind.

Bait nucleic acid (UG)₁₂ is divalent bait nucleic acid which is composed of 2'-O-methyl-RNA nucleosides and to which two TDP-43 proteins can bind, has a structure in which two (UG)₆ sequences are linked via an internucleoside bond, and does not comprise a spacer.

Bait nucleic acid A₁₂ is divalent bait nucleic acid composed of 2'-O-methyl-RNA nucleosides, comprising a (UG)₆ sequence to which one TDP-43 protein can bind at each of the 5' end side and the 3' end side of a nucleic acid strand, and comprising a A₁₂ sequence as a spacer to which TDP-43 cannot bind.

Bait nucleic acid A(L)₁₂ is divalent bait nucleic acid comprising a (UG)₆ sequence which is composed of 2'-O-methyl-RNA nucleosides and to which one TDP-43 protein can bind at each of the 5' end side and the 3' end side of a nucleic acid strand and comprising an A(L)₁₂ sequence composed of locked nucleic acids (LNAs) as a spacer to which TDP-43 cannot bind in a middle region between the (UG)₆ sequences.

Bait nucleic acid A₁₂/t₁₂ is divalent bait nucleic acid consisting of a double-stranded complex, in which one nucleic acid strand has the same configuration as that of the bait nucleic acid A₁₂ above, and the other nucleic acid strand comprises a t₁₂ sequence composed of deoxyribonucleosides.

All the above-described nucleic acid agents were synthesized by GeneDesign Inc., and Hokkaido System Science Co., Ltd., on consignment.

### <Example 2: In vitro aggregation assay>

### (Purpose)

The bait nucleic acids produced in Example 1 are investigated in vitro for the suppression effect against the aggregation of TDP-43 protein.

### (Method and Results)

### (1) Preparation of brain extract comprising TDP-43 protein

A brain extract comprising endogenous TDP-43 protein was prepared by the following method. A striatum extirpated from wild-type mice (C57BL/6J, 5 to 8 weeks old, male) without perfusion with a fixative was homogenized in a H buffer (50 mM HEPES pH7.4, 150 mM NaCl, 10% glycerol, 1% triton X-100, 5 mM MgCl₂, 1 mM EGTA, protease inhibitors, phosphatase inhibitors) in an amount 10 times the brain mass. The homogenize was performed on ice at 900 rpm using a Potter-elvehjem grinder by 10 reciprocating movements. The homogenizing liquid was centrifuged at 4°C and 20,000 × g for 30 min, and then the supernatant was collected. The supernatant was adjusted to 3 mg/mL to be used as a brain extract used for the following in vitro aggregation assay.

### (2) In vitro aggregation assay

The overview of the in vitro aggregation assay is shown in Figure 2. Each bait nucleic acid produced in Example 1 was added to the brain extract prepared in (1) above, and then shaken at 37°C and 1,800 rpm for 3 hours to give a final concentration of 0.2 µM for the (UG)₆ which is monovalent bait nucleic acid and to give a final concentration of 0.1 µM for the other divalent bait nucleic acids. A sample to which no bait nucleic acid was added was also shaken as a negative control. Thereafter, centrifugation was performed at 4°C and 16,000 × g for 30 minutes for separation into two fractions of the supernatant and the precipitate. For the two fractions, TDP-43 protein was detected by Western blotting using a polycronal TDP-43 antibody (12892-1-AP) from Proteintech Group, Inc. (Rosemont, IL), and then the band intensity was quantified using a densitometer to measure the amount of TDP-43 protein comprised in each fraction. The ratio of the TDP-43 protein in the precipitated fraction to the sum of the supernatant fraction and the precipitated fraction was calculated as the aggregation rate.

### (Results)

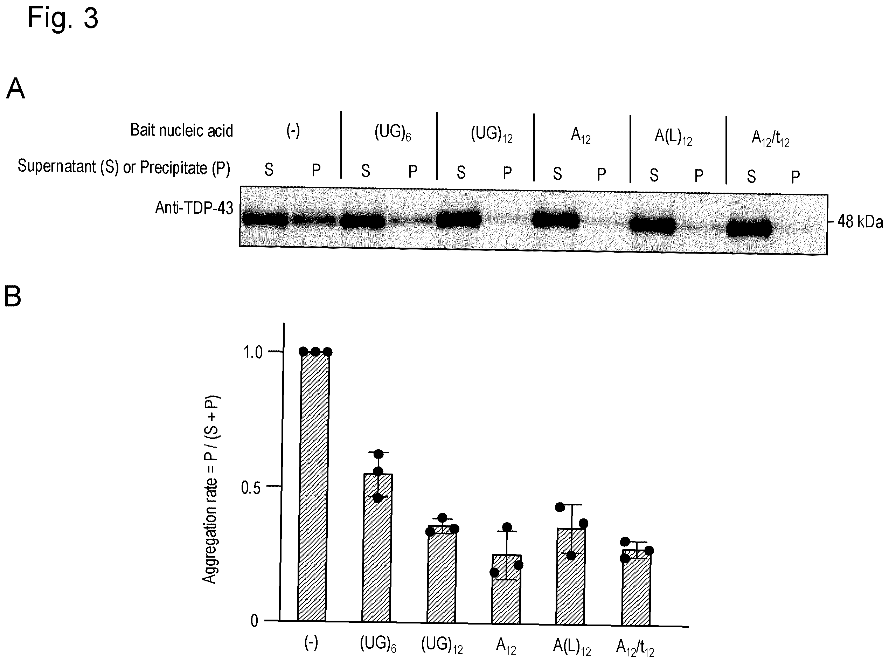

Figure 3 shows the results of examining the TDP-43 aggregation inhibitory effect of each bait nucleic acid by the above-described in vitro aggregation assay. A₁₂, A(L)₁₂, and A₁₂/t₁₂ which are divalent bait nucleic acids comprising a spacer exhibited a stronger aggregation inhibitory effect than that of the (UG)₆ which is monovalent bait nucleic acid and exhibited the aggregation inhibitory effect equal to or higher than that of (UG)₁₂ which is divalent bait nucleic acid comprising no spacer.

### <Example 3: Investigation of aggregation suppression effect against 6M-mutant TDP-43 protein>

### (Purpose)

The aggregation suppression effect against mutant TDP-43 protein which does not form a dimer is investigated.

### (Method and Results)

In this Example, an in vitro aggregation assay was performed using mutant TDP-43 protein having a sextuple mutation (E14A/E17A/E21A/Q34A/R52A/R55A) and deficient in dimer formation ability (hereinafter referred to as "6M-mutant TDP-43 protein "). It is known that this sextuple mutation (E14A/E17A/E21A/Q34A/R52A/R55A) does not affect the folding of the N-terminal domain in TDP-43 protein, but inhibits multimerization (Afroz, T, et al., Nat Commun, 2017, 8, 45). It is considered that a 25 kDa fragment or a 35 kDa fragment deleted in the N-terminal domain, S48 phosphorylated TDP-48 protein, and the like are also considered to be suppressed or deficient in polymer formation ability as in the sextuple mutant.

6M-mutant TDP-43 protein was prepared by in vitro translation. DNA encoding human TDP-43 protein was introduced into a pcDNA3.1(+) vector and the sextuple mutation (E14A/E17A/E21A/Q34A/R52A/R55A) was introduced by site-directed mutagenesis using PCR, thereby producing an expression vector of 6M-mutant TDP-43 protein. 6M-mutant TDP-43 protein was produced using the expression vector and an in vitro translation kit (TNT T7 Quick-Coupled Transcription/Translation System; Promega) using an extract derived from rabbit erythrocytes.

Divalent bait nucleic acid was mixed with the 6M-mutant TDP-43 protein to give a final concentration of 0.1 µM. Similarly, monovalent bait nucleic acid (UG)₆ was mixed to give a final concentration of 0.2 µM. A sample to which no bait nucleic acid was added was used as a negative control. The shaking and centrifugation were carried out in the same manner as in Example 2, the amounts of the TDP-43 proteins in the supernatant fraction and the precipitated fraction were quantified, and the aggregation rate was evaluated.

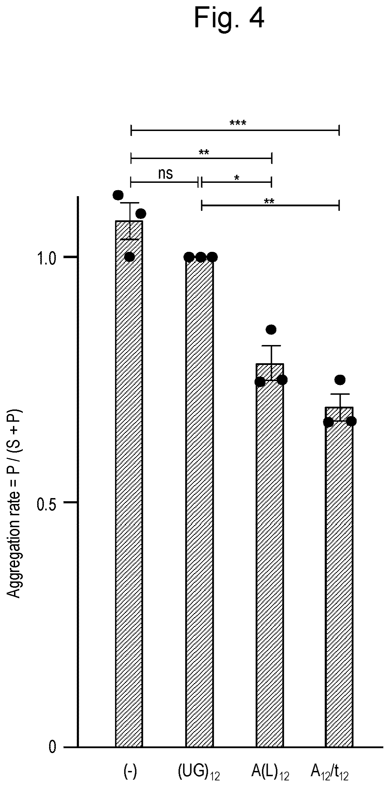

The results are shown in Figure 4. (UG)₁₂ which is divalent bait nucleic acid comprising no spacer did not exhibit a significant aggregation suppression effect compared with that of the negative control to which no bait nucleic acid was added. In contrast thereto, A(L)₁₂ and A₁₂/t₁₂ which are divalent bait nucleic acids comprising a spacer exhibited an aggregation suppression effect, and particularly A₁₂/t₁₂ exhibited the strongest effect. This result has revealed that the aggregation suppression effect against the mutant TDP-43 protein which does not form a dimer is achieved by positioning a spacer between two TDP-43 binding sequences in divalent bait nucleic acid. The 25 kDa fragment or the 35 kDa fragment deleted in the N-terminal domain, the S48 phosphorylated TDP-48 protein, and the like are suppressed or deficient in polymer formation ability as in the sextuple mutation, and therefore the nucleic acid agent of the present invention is considered to have a noticeable aggregation suppression effect regardless of whether ALS is familial or sporadic.

### <Example 4: Evaluation of the presence ratio of monomer and dimer of TDP-43 protein>

### (Purpose)

The presence ratio of a TDP-43 protein dimer to a monomer in the presence of various bait nucleic acids produced in Example 1 is evaluated.

### (Method and Results)

A solution comprising wild-type (WT) TDP-43 protein and 6M-mutant TDP-43 protein expressed by in vitro translation was diluted 2-fold with the H buffer described above, various bait nucleic acids were added to give a final concentration of 0.1 µM for the monovalent bait nucleic acid (UG)₆ and a final concentration of 0.05 µM for the divalent bait nucleic acid, and the mixture was gently shaken at 37°C for 30 minutes. Next, N,N'-Disuccinimidyl glutarate (DSG, Wako) was added to give a final concentration of 1 mM, and the mixture was shaken at room temperature for 30 minutes to carry out a bridging reaction. Thereafter, 20 mM Tris-HCl pH 8.0 was added and the mixture was shaken at room temperature for 15 minutes to terminate the bridging reaction. The bridged nucleic acid-TDP-43 protein complex was detected by Western blotting, and the intensities of the bands corresponding to a monomer and a dimer were quantified to evaluate the ratio of dimers to monomers.

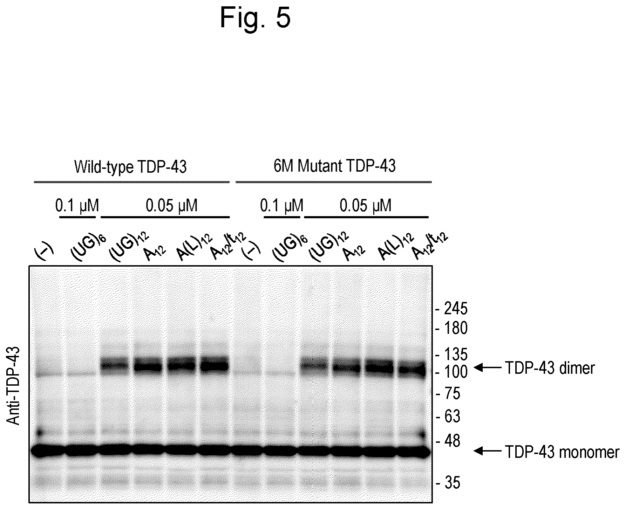

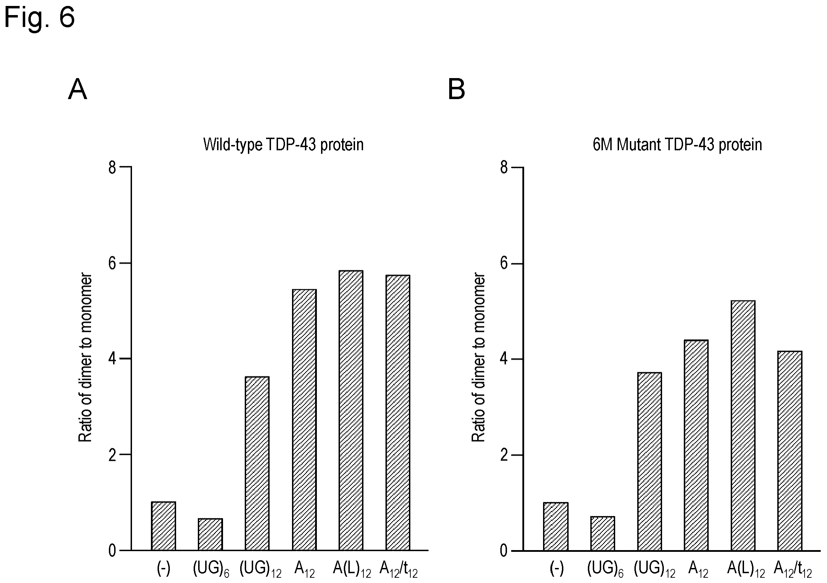

The results are shown in Figures 5 and 6. When no bait nucleic acid was added or when (UG)₆ which is monovalent bait nucleic acid was added, the bands corresponding to TDP-43 monomers were mainly detected for both the wild-type (WT) TDP-43 protein and the 6M-mutant TDP-43 protein, and the ratio of dimers was extremely low. On the other hand, when (UG)₁₂, A₁₂, A(L)₁₂, and A₁₂/t₁₂ which are divalent bait nucleic acids were added, the ratio of the bands corresponding to TDP-43 dimers was found to be high for both the wild-type (WT) TDP-43 protein and the 6M-mutant TDP-43 protein.

The contrast to the result that a dimer was hardly formed in (UG)₆ which is monovalent bait nucleic acid suggested that the two (UG)₆ sequences in the divalent bait nucleic acid each do not independently induce the formation of a dimer, but that the two (UG)₆ sequences cooperate to assist the formation of a dimer. Therefore, in the case where divalent bait nucleic acid was used, it is considered that the bait nucleic acid and the TDP-43 protein are considered to bind to each other in a 1:2 ratio to form a complex.

Further, the ratio of dimers when A₁₂, A(L)₁₂, and A₁₂/t₁₂ were added was higher than that when (UG)₁₂ was added, which has revealed that the ratio of the TDP-43 dimer is increased by positioning a spacer between the two TDP-43 binding sequences in the divalent bait nucleic acid, and has revealed that the introduction of the spacer enhances the above-described cooperative action between the two (UG)₆ sequences (Figure 6).

### <Example 5: Effects of spacer other than nucleic acid>

### (Purpose)

Divalent bait nucleic acid comprising two TDP-43 binding sequences and a spacer other than nucleic acid positioned between the TDP-43 binding sequences was produced, and the aggregation suppression effect against TDP-43 protein is investigated.

### (Method and Results)

### (1) Production of nucleic acid

Divalent bait nucleic acid capable of binding TDP-43 proteins to positions separated from each other by binding the TDP-43 protein binding sequence, to which one molecule of TDP-43 protein can bind, to both ends of a spacer was produced using a spacer other than nucleic acid. Divalent bait nucleic acids produced in this Example are as follows.

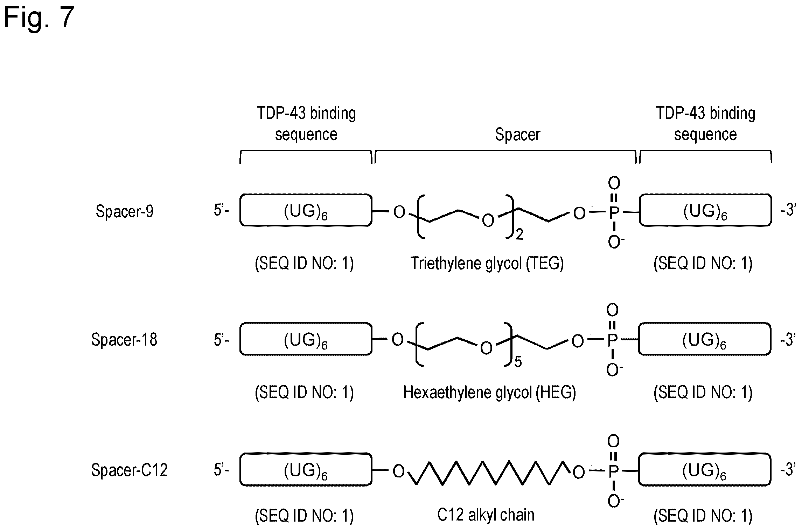

The structure of divalent bait nucleic acid comprising the (UG)₆ sequence which is composed of 2'-O-methyl-RNA nucleosides and to which one TDP-43 protein can bind at both ends of triethylene glycol (TEG) (hereinafter referred to as "Spacer-9") is shown in the following formula (VII) and Figure 7. 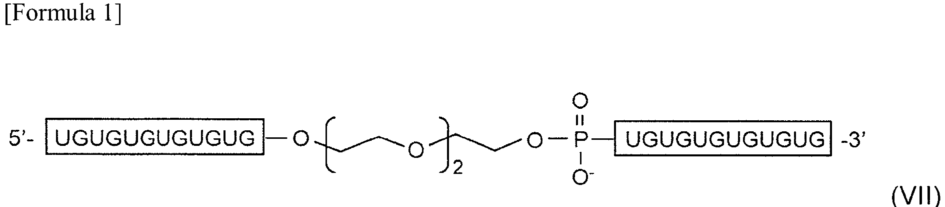 (wherein U and G represent 2'-O-methyl-RNA.)

The structure of divalent bait nucleic acid comprising the (UG)₆ sequence which is composed of 2'-O-methyl-RNA nucleosides and to which one TDP-43 protein can bind at both ends of hexaethylene glycol (HEG) (hereinafter referred to as "Spacer-18") is shown in the following formula (VIII) and Figure 7. 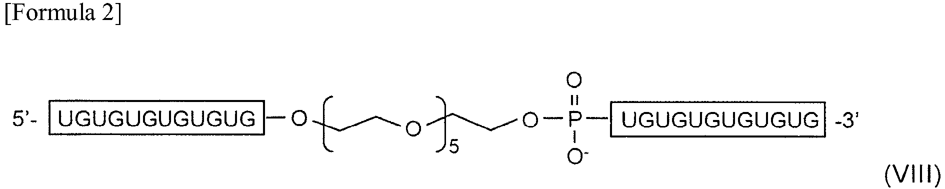 (wherein U and G represent 2'-O-methyl-RNA.)

The structure of divalent bait nucleic acid comprising the (UG)₆ sequence which is composed of 2'-O-methyl-RNA nucleosides and to which one TDP-43 protein can bind at both ends of an alkylene chain having 12 carbon atoms (C12 alkylene chain) (hereinafter referred to as "Spacer-18") is shown in the following formula (IX) and Figure 7. 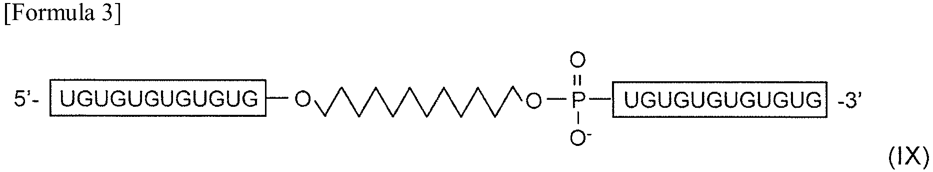 (wherein U and G represent 2'-O-methyl-RNA.)

All the above-described nucleic acid agents were synthesized by GeneDesign Inc., and Hokkaido System Science Co., Ltd., on consignment.

### (2) In vitro aggregation assay

An in vitro aggregation assay was performed in the same manner as in Example 2. Specifically, bait nucleic acid was added to a brain extract to give a final concentration of 0.1 µM. Thereafter, shaking and centrifugation were carried out, the amounts of the TDP-43 proteins in the supernatant fraction and the precipitated fraction were quantified, and the aggregation rate was evaluated in the same manner as in Example 2. A sample to which no bait nucleic acid was added was used as a negative control.

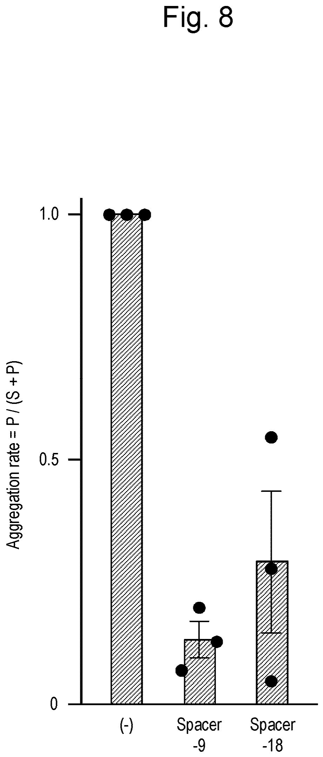

The results are shown in Figure 8. The Spacer-9 and the Spacer-18 which are divalent bait nucleic acids comprising TEG or HEG as a spacer exhibited the aggregation suppression effect against TDP-43 protein, and particularly the Spacer-9 exhibited the strongest effect.

The results of this Example have revealed that a strong aggregation suppression effect can be obtained even when a spacer other than nucleic acid was used. This result has revealed that the aggregate formation between TDP-43 proteins bound to the TDP-43 binding sequences can be efficiently suppressed by positioning two TDP-43 binding sequences at positions separated from each other via a rigid spacer.

### <Example 6: Further production of divalent bait nucleic acid and trivalent bait nucleic acid>

### (Purpose)

Divalent bait nucleic acids having spacers of various lengths were produced. Bait nucleic acid comprising three TDP-43 binding sequences (hereinafter referred to as "trivalent bait nucleic acid") is produced. Further, as complex-type bait nucleic acid, nucleic acid complexes are produced which consist of two nucleic acid strands and in which each nucleic acid strand comprises a TDP-43 binding sequence and an adapter sequence that enables the formation of a double strand between the nucleic acid strands (hereinafter referred to as "divalent bait nucleic acid complex").

### (Method and Results)

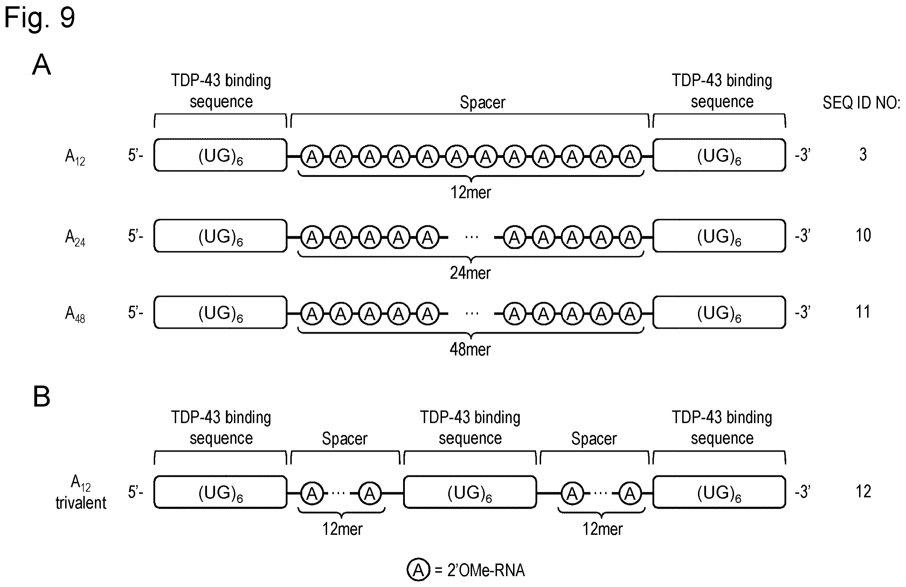

The structures of the bait nucleic acids produced in this Example are shown in Table 2 below and in Figure 9.

**[Table 2]**

| Table 2: Structure of bait nucleic acid | | |
|---|---|---|
| Name of bait nucleic acid | Sequence (5'-3') | SEQ ID NO |
| (UG)₁₂ | UGUGUGUGUGUG UGUGUGUGUGUG | 2 |
| A₁₂ | UGUGUGUGUGUG AAAAAAAAAAAA UGUGUGUGUGUG | 3 |
| A₂₄ | UGUGUGUGUGUG AAAAAAAAAAAA AAAAAAAAAAAA UGUGUGUGUGUG | 10 |
| A₄₈ | UGUGUGUGUGUG AAAAAAAAAAAA AAAAAAAAAAAA AAAAAAAAAAAA AAAAAAAAAAAA UGUGUGUGUGUG | 11 |
| A₁₂ trivalent | UGUGUGUGUGUG AAAAAAAAAAAA UGUGUGUGUGUG AAAAAAAAAAAA UGUGUGUGUGUG | 12 |

| | | |
|---|---|---|
| **Upper** case letter: 2'-O-methyl-RNA; Internucleoside linkages are all phosphodiester linkages. | | |

Bait nucleic acid (UG)₁₂ and A₁₂ are the same as the bait nucleic acid (UG)₁₂ and A₁₂, respectively, produced in Example 1.

Bait nucleic acid A₂₄ is divalent bait nucleic acid comprising two (UG)₆ sequences composed of 2'-O-methyl-RNA nucleosides and comprising a spacer consisting of a A₂₄ sequence between the (UG)₆ sequences.

Bait nucleic acid A₄₈ is divalent bait nucleic acid comprising two (UG)₆ sequences composed of 2'-O-methyl-RNA nucleosides and comprising a spacer consisting of a A₄₈ sequence between the (UG)₆ sequences.

Bait nucleic acid A₁₂ trivalent is trivalent bait nucleic acid comprising three (UG)₆ sequences composed of 2'-O-methyl-RNA nucleosides and comprising two spacers consisting of a A₁₂ sequence among the (UG)₆ sequences.

In addition to the above-described bait nucleic acids, the following bait nucleic acids A₃, A₆, and A₉ were produced.

Bait nucleic acid A₃ comprising two (UG)₆ sequences composed of 2'-O-methyl-RNA nucleosides and comprising a spacer consisting of a A₃ sequence between the (UG)₆ sequences.

Bait nucleic acid A₆ comprising two (UG)₆ sequences composed of 2'-O-methyl-RNA nucleosides and comprising a spacer consisting of a A₆ sequence between the (UG)₆ sequences.

Bait nucleic acid A₉ comprising two (UG)₆ sequences composed of 2'-O-methyl-RNA nucleosides and comprising a spacer consisting of a A₉ sequence between the (UG)₆ sequences.

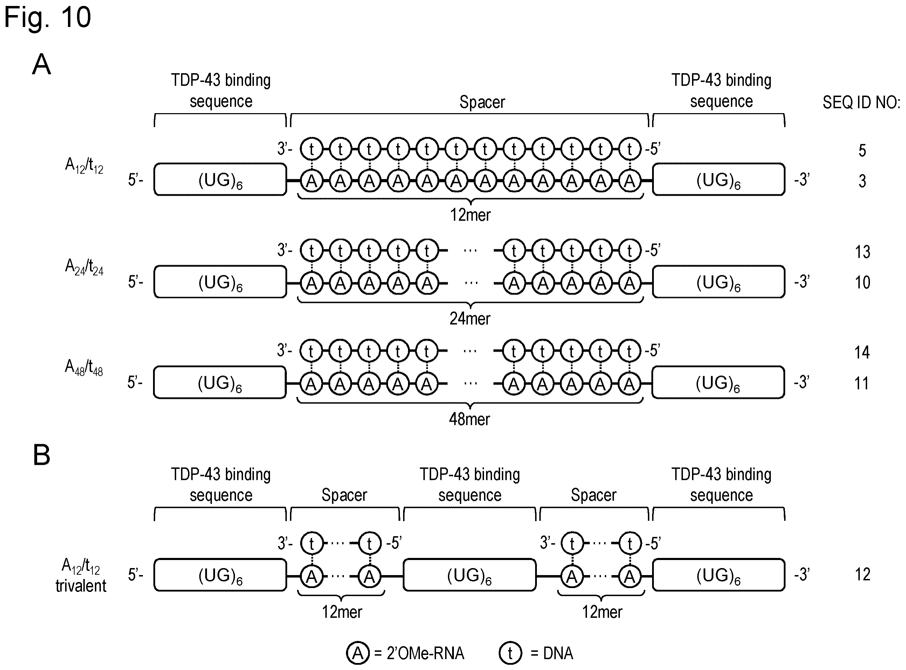

Further, bait nucleic acids were produced which were transformed into a complex with a complementary strand consisting of a t₁₂ sequence, a t₂₄ sequence, or a t₄₈ sequence composed of deoxyribonucleosides so that a spacer moiety in the bait nucleic acid shown in Table 2 can form a double-stranded structure (Figure 10).

Bait nucleic acid A₁₂/t₁₂ is the same as the bait nucleic acid A₁₂/t₁₂ produced in Example 1.

The bait nucleic acid A₂₄/t₂₄ is divalent bait nucleic acid consisting of a double-stranded complex, in which one nucleic acid strand has the same configuration as that of the bait nucleic acid A₂₄, and the other nucleic acid strand is a t₂₄ sequence composed of deoxyribonucleosides.

The bait nucleic acid A₄₈/t₄₈ is divalent bait nucleic acid consisting of a double-stranded complex, in which one nucleic acid strand has the same configuration as that of the bait nucleic acid A₄₈, and the other nucleic acid strand is a t₄₈ sequence composed of deoxyribonucleosides.

The bait nucleic acid A₁₂/t₁₂ is complex-type trivalent bait nucleic acid composed of three nucleic acid strands. In addition to the above-described bait nucleic acid A₁₂ trivalent, two complementary strands consisting of a t₁₂ sequence composed of deoxyribonucleosides are comprised.

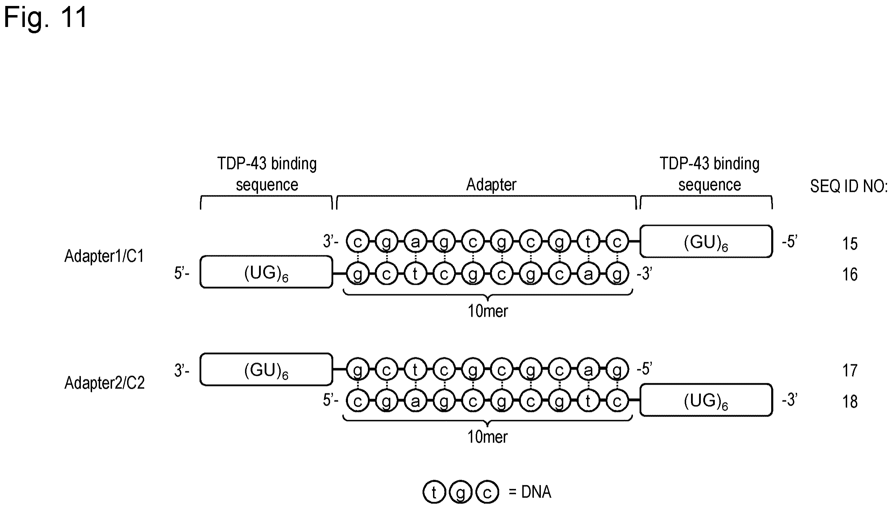

The structures of the divalent bait nucleic acid complexes further produced in this Example are shown in Table 3 below and Figure 11.

**[Table 3]**

| Table 3: Structure of bait nucleic acid complex | | | |
|---|---|---|---|
| Name of bait nucleic acid | | Sequence (5'-3') | SEQ ID NO |
| Adaper1 /C1 | Adapter1 strand | UGUGUGUGUGUG ctgcgcgagc | 15 |
| | C1 strand | UGUGUGUGUGUG gctcgcgcag | 16 |
| Adaper2 /C2 | Adapter2 strand | gacgcgctcg UGUGUGUGUGUG | 17 |
| | C2 strand | cgagcgcgtc UGUGUGUGUGUG | 18 |

| | | | |
|---|---|---|---|
| Upper case letter: 2'-O-methyl-RNA; Lower-case letter: DNA; Internucleoside linkages are all phosphodiester linkages. | | | |

A divalent bait nucleic acid complex Adapter1/C1 is composed of an Adapter1 strand and a C1 strand. The Adapter1 strand and the C1 strand each comprise, from the 5' side, a (UG)₆ sequence composed of 2'-O-methyl-RNA nucleosides and an adapter sequence having 10 bases in length composed of deoxyribonucleosides. The adapter sequences in the Adapter1 strand and the C1 strand consist of complementary base sequences.

A divalent bait nucleic acid complex Adapter2/C2 is composed of an Adapter2 strand and a C2 strand. The Adapter2 strand and the C2 strand each comprise, from the 5' side, an adapter sequence having 10 bases in length composed of deoxyribonucleosides and a (UG)₆ sequence composed of 2'-O-methyl-RNA nucleosides. The adapter sequences in the Adapter2 strand and the C2 strand consist of complementary base sequences.

All the above-described nucleic acid agents were synthesized by GeneDesign Inc., and Hokkaido System Science Co., Ltd., on consignment.

### <Example 7: In vitro aggregation assay and quantitative analysis of dimer>

### (Purpose)

The bait nucleic acids produced in Example 6 are investigated in vitro for the suppression effect against the aggregation of TDP-43 protein. A TDP-43 protein dimer is quantified in the presence of each bait nucleic acid.

### (Method and Results)

### (1) In vitro aggregation assay

An in vitro aggregation assay was performed in the same manner as in Example 2. Specifically, each bait nucleic acid produced in Example 6 was added to a brain extract to give a final concentration of 0.05 µM. Thereafter, shaking and centrifugation were carried out in the same manner as in Example 2, the amounts of the TDP-43 proteins in the supernatant fraction and the precipitated fraction were quantified, and the aggregation rate was evaluated. A sample to which no bait nucleic acid was added was used as a negative control.

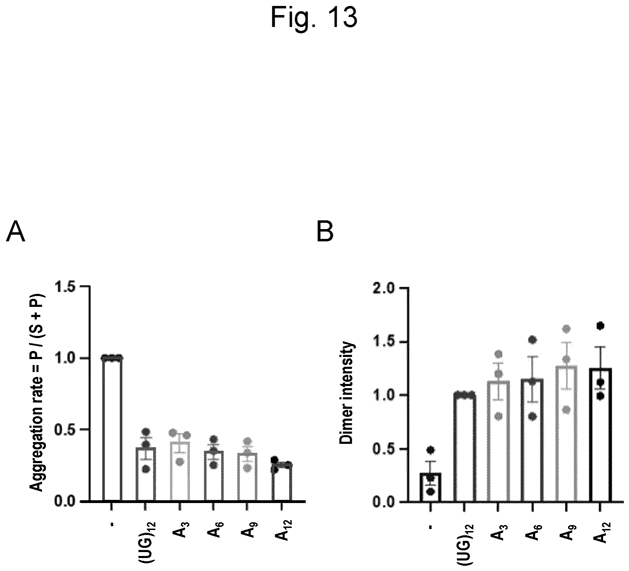

The results are shown in Figures 12A and 13A. It has been revealed that A₁₂ and A₂₄ tend to be more excellent in the aggregation suppression effect than (UG)₁₂ and A₄₈ (Figure 12A), and that A₁₂ tends to have a higher aggregation suppression effect than that of A₃, A₆, and A₉ (Figure 13A). Further, it has also been revealed that the A₁₂ trivalent is more excellent in the aggregation suppression effect than A₄₈, as in A₁₂ and A₂₄ (Figure 12A).

### (2) Quantification of TDP-43 protein dimer

A brain extract of a wild-type mouse was prepared in the same manner as in "(1) Preparation of brain extract comprising TDP-43 protein" in Example 2. To this brain extract, various bait nucleic acids were added to give a final concentration of 0.05 µM, and the mixture was gently shaken at 37°C for 30 minutes. Next, N,N'-disuccinimidyl glutarate (DSG, Wako) was added to give a final concentration of 1 mM, and the mixture was shaken at room temperature for 30 minutes to carry out a bridging reaction. Thereafter, Tris-HCl pH 8.0 was added to give a final concentration of 20 mM, and the solution was shaken at room temperature for 15 minutes to terminate the bridging reaction. The bridged nucleic acid-TDP-43 protein complex was detected by Western blotting and the intensity of the band corresponding to a dimer was quantified to measure the abundance of a dimer.

The results are shown in Figures 12B and 13B. It has been revealed that the amount of the TDP-43 dimer tends to be larger in the presence of A₁₂ and A₂₄ than in the presence of (UG)₁₂ and A₄₈ (Figure 12B), and the amount of the TDP-43 dimer tends to be larger in the presence of A₁₂ than in the presence of A₃ and A₆ (Figure 13B). Further, it has been revealed that the amount of the TDP-43 dimer is much higher in the presence of the A₁₂ trivalent than in the presence of A₁₂ and A₂₄ (Figure 12B).

The results of (1) and (2) above have revealed that the bait nucleic acid having a spacer consisting of nucleic acid strands having 12 to 24 bases in length particularly has a high effect.

### (3) Correlation analysis

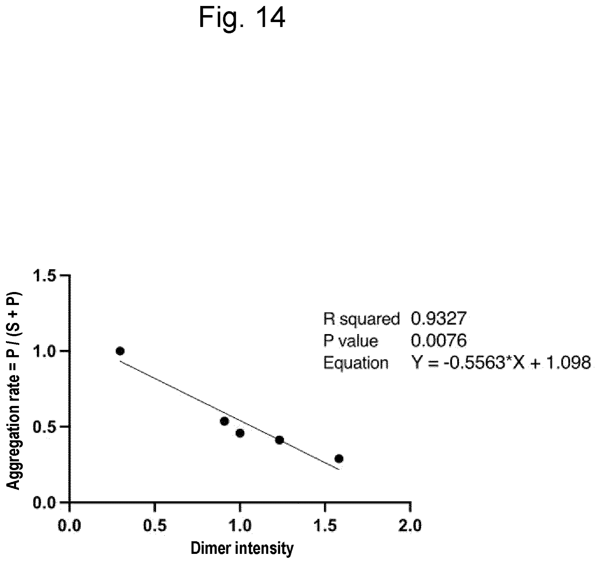

Based on the above-described results of (1) and (2), the presence or absence of a correlation between the quantification value of the dimer and the aggregation rate in the in vitro aggregation assay was investigated. FIG. 14 shows the results of plotting the quantitative results of the dimer on the horizontal axis and the aggregation rate on the vertical axis for (UG)₁₂, A₁₂, A₂₄, A₄₈, and A₁₂ trivalent. The coefficient of determination (R² value) was 0.9327, revealing that there is an extremely high correlation. This result reveals that bait nucleic acids which increase the amount of a dimer has a higher aggregation suppression effect.

### <Example 8: Further production of bait nucleic acid comprising spacer other than nucleic acid> (Purpose)

Further divalent bait nucleic acids comprising two TDP-43 binding sequences and a spacer other than nucleic acid positioned between the TDP-43 binding sequences were produced.

### (Method and Results)

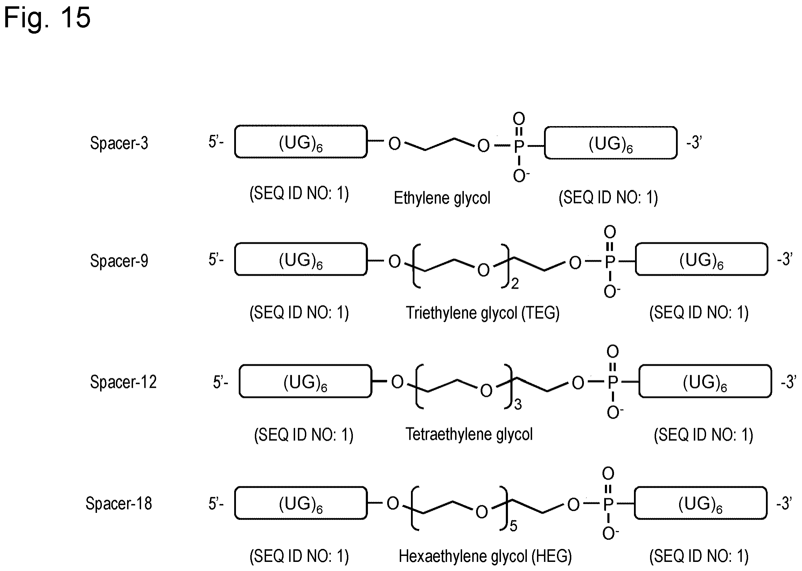

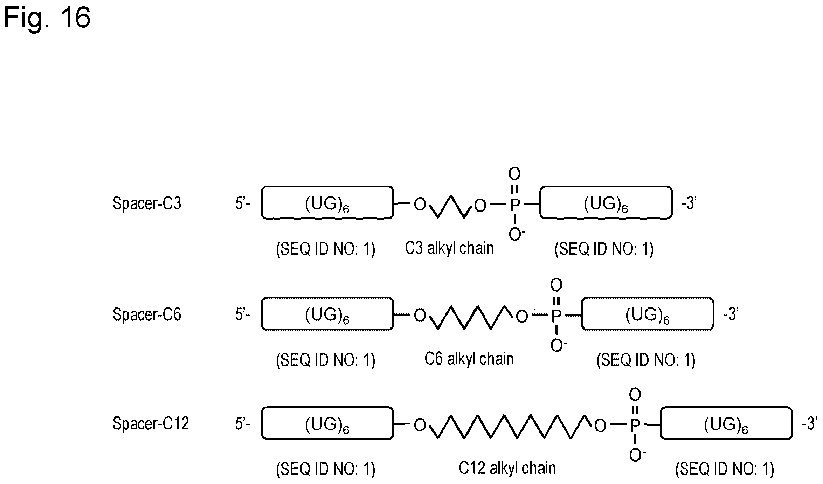

The structures of bait nucleic acids produced in this Example are shown in Figures 15 and 16. The structures of the divalent bait nucleic acids produced in this Example are as follows.

A bait nucleic acid Spacer-3 is divalent bait nucleic acid comprising two (UG)₆ sequences composed of 2'-O-methyl-RNA nucleosides and a spacer consisting of an ethylene glycol group positioned between the (UG)₆ sequences. The structure of the bait nucleic acid Spacer-3 is shown in Formula (X) below. 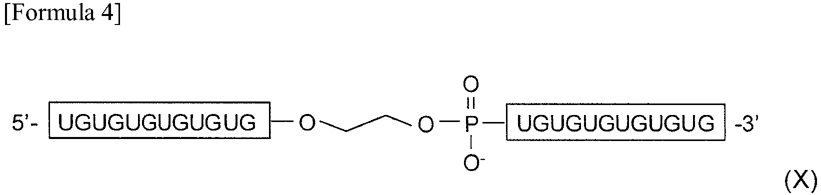 (wherein U and G represent 2'-O-methyl-RNA.)

A bait nucleic acid Spacer-9 is divalent bait nucleic acid comprising two (UG)₆ sequences composed of 2'-O-methyl-RNA nucleosides and a spacer consisting of a triethylene glycol group positioned between the (UG)₆ sequences. The structure of the bait nucleic acid Spacer-9 is as shown in Formula (VII) above.

A bait nucleic acid Spacer-12 is divalent bait nucleic acid comprising two (UG)₆ sequences composed of 2'-O-methyl-RNA nucleosides and a spacer consisting of a tetraethylene glycol group positioned between the (UG)₆ sequences. The structure of the bait nucleic acid Spacer-12 is shown in Formula (XI) below. 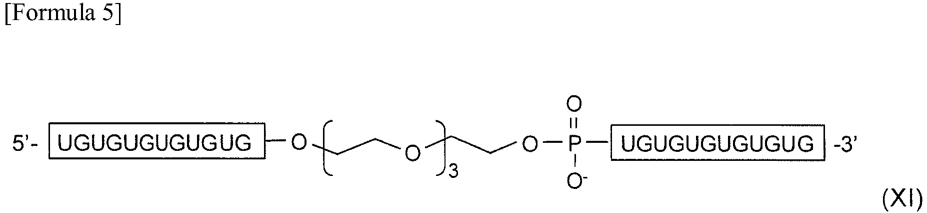 (wherein U and G represent 2'-O-methyl-RNA.)

A bait nucleic acid Spacer-18 is divalent bait nucleic acid comprising two (UG)₆ sequences composed of 2'-O-methyl-RNA nucleosides and a spacer consisting of a hexaethylene glycol group positioned between the (UG)₆ sequences. The structure of the bait nucleic acid Spacer-18 is as shown in Formula (VIII) above.

A bait nucleic acid Spacer-C3 is divalent bait nucleic acid comprising two (UG)₆ sequences composed of 2'-O-methyl-RNA nucleosides and a spacer consisting of an alkylene chain having 3 carbon atoms (C3 alkylene chain) positioned between the (UG)₆ sequences. The structure of the bait nucleic acid Spacer-C3 is shown in the following formula (XII). 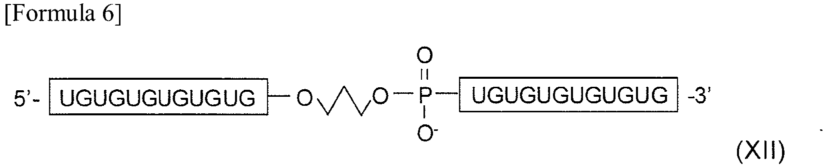 (wherein U and G represent 2'-O-methyl-RNA.)

A bait nucleic acid Spacer-C6 is divalent bait nucleic acid comprising two (UG)₆ sequences composed of 2'-O-methyl-RNA nucleosides and a spacer consisting of an alkylene chain having 6 carbon atoms (C6 alkylene chain) positioned between the (UG)₆ sequences. The structure of the bait nucleic acid Spacer-C6 is shown in the following formula (XII). 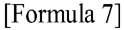 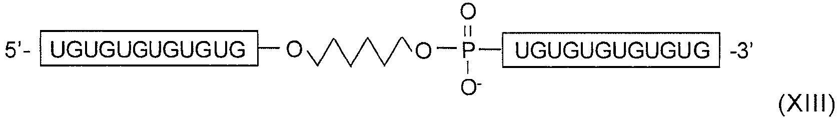 (wherein U and G represent 2'-O-methyl-RNA.)

A bait nucleic acid Spacer-C12 is divalent bait nucleic acid comprising two (UG)₆ sequences composed of 2'-O-methyl-RNA nucleosides and a spacer consisting of an alkylene chain having 12 carbon atoms (C12 alkylene chain) positioned between the (UG)₆ sequences. The structure of the bait nucleic acid Spacer-C12 is as shown in Formula (IX) above.

All the above-described nucleic acid agents were synthesized by GeneDesign Inc., and Hokkaido System Science Co., Ltd., on consignment.

### <Example 9: In vitro aggregation assay and quantification of dimer>

### (Purpose)

The bait nucleic acids produced in Example 8 are investigated in vitro for the suppression effect against the aggregation of TDP-43 protein. A TDP-43 protein dimer is quantified in the presence of each bait nucleic acid.

### (Method and Results)

The bait nucleic acids produced in Example 8 were subjected to an in vitro aggregation assay and the quantification of a dimer in the same manner as in Example 7.

The results of the bait nucleic acids (UG)₁₂, Spacer-3, Spacer-9, Spacer-12, and Spacer-18 are shown in Figure 17. It has been revealed that the Spacer-3, Spacer-9, and Spacer-12 are more excellent in the aggregation suppression effect than (UG)₁₂ and the Spacer-18 (Figure 17A) and that the amount of the TDP-43 dimer is larger in the presence of the Spacer-3, Spacer-9, and Spacer-12 than in the presence of (UG)₁₂ and the Spacer-18 (Figure 17B). These results have revealed that the bait nucleic acid having a spacer consisting of one to four ethylene glycol groups has a particularly high effect.

The results of the bait nucleic acids (UG)₁₂, Spacer-C3, Spacer-C6, and Spacer-C12 are shown in Figure 18. It has been revealed that the Spacer-C3 and Spacer-C6 are excellent in the aggregation suppression effect than (UG)₁₂ and the Spacer-C12 (Figure 18A) and that the amount of the TDP-43 dimer is larger in the presence of the Spacer-C3 and Spacer-C6 than in the presence of (UG)₁₂ and the Spacer-C12 (Figure 18B). These results have revealed that the bait nucleic acid having a spacer consisting of a C₃₋₆ alkylene group has a particularly high effect.

### <Example 10: Aggregation suppression effect against 6M-mutant TDP-43 protein>

### (Purpose)

The bait nucleic acids and the bait nucleic acid complexes produced in Examples 6 and 8 are investigated for the aggregation suppression effect against 6M-mutant TDP-43 protein.

### (Method and Results)

In this Example, an in vitro aggregation assay was performed in the same manner as in Example 3 using 6M-mutant TDP-43 protein having a sextuple mutation (E14A/E17A/E21A/Q34A/R52A/R55A) and deficient in dimer formation ability. Specifically, among the bait nucleic acids produced in Examples 6 and 8, the monovalent bait nucleic acid (UG)₆ was mixed with 6M-mutant TDP-43 protein to give a final concentration of 0.2 µM, and the other bait nucleic acids were mixed with 6M-mutant TDP-43 protein to give a final concentration of 0.1 µM. Shaking and centrifugation were carried out and the amounts of the TDP-43 proteins in the supernatant fraction and the precipitated fraction were quantified to evaluate the aggregation rate.

The results are shown in Figure 19. It has been revealed that the bait nucleic acids A₁₂, A₂₄, A₄₈, and A₁₂ trivalent are more excellent in the aggregation suppression effect than the bait nucleic acid (UG)₁₂ (Figure 19A), that the bait nucleic acids A₁₂/t₁₂, A₂₄/t₂₄, A₄₈/t₄₈, and A₁₂/t₁₂ trivalent are more excellent in the aggregation suppression effect than the bait nucleic acid (UG)₁₂ (Figure 19B), and that the bait nucleic acid complex Adapter1/C1 is more excellent in the aggregation suppression effect than the bait nucleic acid (UG)₁₂ (Figure 19C).

### <Example 11: Toxicity evaluation>

### (Purpose)

The acute toxicity and the chronic toxicity were evaluated when the bait nucleic acid A₁₂ and the bait nucleic acid Spacer-9 were applied by intraventricular administration to a mouse.

### (Method and Results)

### (1) Acute toxicity evaluation

The acute toxicity evaluation was carried out according to the method described in the literature (Jia C. et al., Mol Ther Nucleic Acids, 2022, 31:182-196.). Specifically, 5-week-old male C57BL/6J mice were fixed to a brain stereotaxic apparatus under isoflurane anesthesia, the skin was cut open, and the skull was perforated using a 1 mm diameter drill. A Hamilton syringe was filled with PBS (control), the bait nucleic acid A₁₂, the bait nucleic acid Spacer-9, or Toxic Oligo (positive control) which is a control nucleic acid exhibiting acute toxicity. Through the perforated site, a needle was inserted approximately 3 mm deep for intracerebroventricular administration. Then, the skin was sutured with a nylon thread. The dose of the nucleic acid agent was 100 µg.

The mice after the administration were evaluated for central nervous system toxicity. Specifically, the behavior was evaluated using the scoring system which evaluates the behaviors belonging to five categories described in the above-described literature and death at 1 hour and 3 hours after the administration. For each behavior evaluation item, a normal behavior is given 0 points, and higher toxicity is given a higher score. The total value of the scores for the five categories and death is defined as a toxicity score (0 to 22 points).

The results are shown in Figure 20A. The positive control Toxic Oligo exhibited a toxicity score of about 3 one hour after the administration and about 8 three hours after the administration. On the other hand, it has been revealed that the bait nucleic acid A₁₂ and the Spacer-9 exhibit no acute toxicity at either 1 hour or 3 hours after the administration.

### (2) Chronic toxicity evaluation

The motor cortex was extirpated from the mice at 7 days after the intracerebroventricular administration in (1) above. Using a Fast Gene RNA Basic kit (NIPPON Genetics Co, Ltd.), RNA was extracted from the extirpated motor cortex. A cDNA was synthesized using PrimeScript RT Master Mix (TaKaRa) in accordance with the protocol. Next, the resulting cDNA template was used to perform quantitative RT-PCR, whereby the expression levels of TNF-α mRNA, IL-1 β mRNA, GFAP mRNA, and GAPDH mRNA (internal standard gene) were measured. Quantitative RT-PCR was carried out with TaqMan (Roche Applied Science). Primers used in quantitative RT-PCR were products designed and manufactured by Thermo Fisher Scientific Inc. (formerly known as Life Technologies Corp.). Amplification conditions (temperature and time) were as follows: the cycle, 95°C for 10 seconds, 60°C for 30 seconds, and 72°C for 1 second, was repeated 45 times. The ratio of the expression levels of TNF-α mRNA, IL-1β mRNA, and GFAP mRNA to the expression level of GAPDH mRNA (internal standard gene) was calculated, and a value standardized with respect to the value of the PBS-treated group was determined as a relative Mapt mRNA level.

The results are shown in Figures 20B to 20D. In the mice to which the bait nucleic acid A₁₂ and the bait nucleic acid Spacer-9 were administered, no significant changes were observed in the expression levels of inflammatory markers of TNF-α mRNA and IL-1β mRNA and a gliosis marker of GFAP mRNA compared with the mice to which PBS was administered. These results have revealed that both the bait nucleic acid A₁₂ and the Spacer-9 have low toxicity.

### <Example 12: Analysis of survival time>

### (Purpose)

It is investigated that the single-dose administration of the bait nucleic acid A₁₂ and the bait nucleic acid Spacer-9 to the mouse ventricle increases the survival time of an amyotrophic lateral sclerosis (ALS) model mouse.

In this Example, rNLS mice described in the literature (Waker A. K., et al., Acta Neuropathol., 2015, 130(5):643-660.) are used as an ALS model mouse. In the rNLS mice, the expression of a nuclear localization signal (ΔNLS)-deficient human TDP-43 protein (hTDP-43ΔNLS) is induced under a condition in which doxycycline (Dox) is not administered, and insoluble TDP-43 accumulates in the brain and spinal cord, leading to death.

### (Method and Results)

A vehicle (PBS), the bait nucleic acid A₁₂, or the bait nucleic acid Spacer-9 was intracerebroventricularly administered to 5-week-old rNLS mice raised under a condition in which Dox was administered in the same manner as in Example 11. The dose of the nucleic acid agent is 100 µg, and each treated group is composed of three mice (1 male and 2 females). The Dox administration was continued until the intraventricular administration day. From the intraventricular administration day, the Dox administration was not performed, in order to initiate the induction of the expression of hTDP-43ΔNLS. This date was set as the reference date (0 days Dox-off), and the survival rate of the mice after the reference date was analyzed.

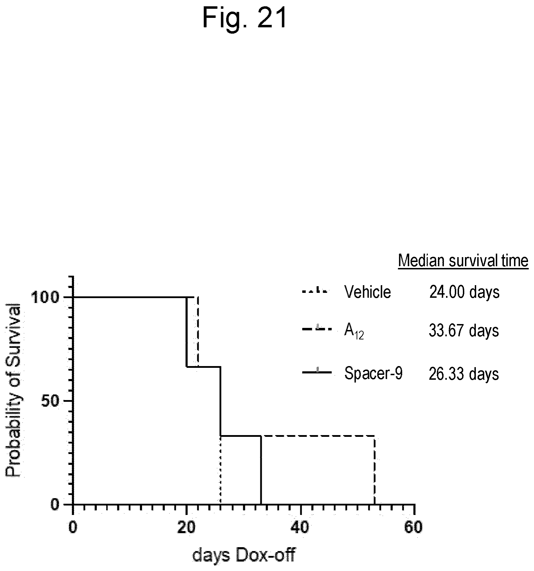

The results are shown in Figure 21. The median survival time of the vehicle-treated group was 24.0 days. In contrast thereto, the median survival time of the Spacer-9-treated group was 26.33 days, and the median survival time of the A₁₂-treated group was about 33.67 days. It has been revealed that particularly the A₁₂-treated group exhibits a significant extension of the survival time.

### <Example 13: Rotarod test>

### (Purpose)

The bait nucleic acid A₁₂ and the bait nucleic acid Spacer-9 are applied by intraventricular administration to the ALS model mice (rNLS mice). A therapeutic effect on hypokinesia of the ALS model mice is investigated by a rotarod test.

### (Method and Results)

A vehicle (PBS), the bait nucleic acid A₁₂, or the bait nucleic acid Spacer-9 was intracerebroventricularly administered to 5-week-old rNLS mice (1 male and 2 females) raised under a condition in which Dox was administered in the same manner and doses as in Example 12. The Dox administration was continued until the intraventricular administration day. Using the intraventricular administration day as the reference point, the rotarod test was carried out before the administration (0 week off-Dox), one week after the administration (1 week off-Dox), and two weeks after the administration (2 weeks off-Dox) to evaluate the motor function.

The rotarod test was carried out by the following method. The mice were placed on a rotor rod apparatus (Ugo Basile 7650), and the rotation speed of the rotor was accelerated from an initial speed of 4 rpm to a final speed of 40 rpm over a period of 300 seconds. This was set as one session, and one training session and two test sessions were performed. Between the sessions, a 30-minute break was set. In the test session, the time from when the mouse was placed on the apparatus until the mouse fell was recorded, with the time recorded as 300 seconds when the mouse had not fallen at the end of the session. The average time of the two test sessions was calculated, and a score was calculated in which the performance at each point was standardized with the performance before the administration as 100.

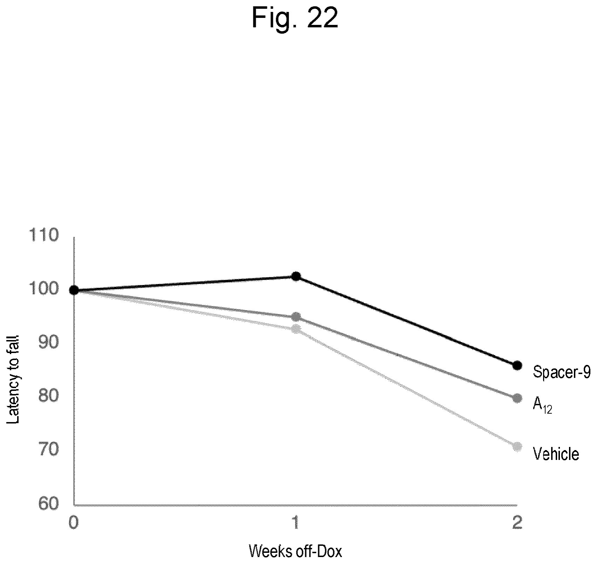

The results are shown in Figure 22. The PBS-treated group exhibited a noticeable decrease in scores and a noticeable decline in motor function 1 week and 2 weeks after the administration compared with the scores before the administration. On the other hand, in the A₁₂-treated group, the motor function decline was significantly mitigated, and, in the Spacer-9-treated group, the mitigation effect on the motor function decline was particularly large. These results have revealed that the bait nucleic acid is effective in maintaining the motor function in the ALS model mouse.

All publications, patents, and patent applications cited herein are incorporated herein by reference in their entirety.

## Claims

1. A multivalent binding nucleic acid agent which binds to TDP-43 protein or a fragment thereof, comprising two or more nucleic acid strands, wherein each of the two or more nucleic acid strands comprises a TDP-43 binding sequence capable of binding to TDP-43 protein or a fragment thereof, and wherein:
(1) the two or more nucleic acid strands are bound via a linker, or
(2) the two or more nucleic acid strands comprises an adapter sequence that enables formation of multiplex strands between the two or more nucleic acid strands.

2. The nucleic acid agent of claim 1, wherein the linker has a rigidity and/or a length that can reduce an aggregate formation by two or more monomeric TDP-43 proteins which are bound to the TDP-43 binding sequences of the two or more nucleic acid strands.

3. The nucleic acid agent of claim 2, wherein the reduction of the aggregate formation is based on a reduced binding between the C-terminal regions of the TDP-43 proteins and/or an increased phase separation thereof.

4. The nucleic acid agent of any one of claims 1 to 3, wherein the linker comprises a nucleic acid, peptide, polyether group, and/or hydrocarbon group.

5. The nucleic acid agent of claim 4, wherein the nucleic acid consists of one or two to 50 nucleosides linked by internucleoside bonds.

6. The nucleic acid agent of claim 5, wherein the nucleoside comprises a natural nucleoside and/or a non-natural nucleoside.

7. The nucleic acid agent of claim 4, wherein the nucleic acid is a peptide nucleic acid.

8. The nucleic acid agent of claim 4, wherein the polyether group is a polyethylene glycol group.

9. The nucleic acid agent of claim 4, wherein the hydrocarbon group is an optionally substituted hydrocarbon group with carbon number 2 to 30.

10. The nucleic acid agent of claim 1, wherein the linker comprises a region consisting of a single-stranded nucleic acid.

11. The nucleic acid agent of claim 10, wherein the region consisting of the single-stranded nucleic acid comprises a hairpin structure.

12. The nucleic acid agent of claim 10 or 11, further comprising a complementary strand which comprises a base sequence complementary to at least part of the region consisting of the single-stranded nucleic acid, wherein the region consisting of the single-stranded nucleic acid and the complementary strand form a multiplex strand structure.

13. The nucleic acid agent of claim 12, wherein the region consisting of the single-stranded nucleic acid comprises a non-complementary base, and/or an insertion sequence and/or a deletion of one or more bases, with respect to the complementary strand.

14. The nucleic acid agent of claim 13, wherein the region consisting of the single-stranded nucleic acid comprises one to three of the non-complementary bases.

15. The nucleic acid agent of claim 13, wherein the insertion sequence consists of one to eight bases.

16. The nucleic acid agent of claim 13, wherein the deletion consists of one to four consecutive bases.

17. The nucleic acid agent of claim 12, wherein the complementary strand comprises a natural nucleoside and/or a non-natural nucleoside.

18. The nucleic acid agent of claim 1, wherein the adaptor sequence is 8 to 50 bases in length.

19. The nucleic acid agent of claim 1, wherein the TDP-43 binding sequence binds to an RNA recognition motif (RRM) of the TDP-43 protein.

20. The nucleic acid agent of claim 1, wherein the TDP-43 binding sequence consists of a repeat sequence represented by the following formula (I):
(X₀ G X₁ X₂ X₃ ... Xₘ)ₙ (I),
wherein X₀ is T or U; X₁, X₂, X₃, ..., and Xₘ are independently any of A, C, G, T, or U, and may be identical or different, or X₁, X₂, X₃, ..., and Xₘ are absent; m is 1 to 10; and n represents a repeat number of 2 to 50.

21. The nucleic acid agent of claim 1, wherein the TDP-43 binding sequence consists of a repeat sequence represented by the following formula (IV):
(X G)ₙ (IV),
wherein X represents T or U, and n is three or more.

22. The nucleic acid agent of claim 21, wherein each of the TDP-43 binding sequence can bind to one TDP-43 protein or a fragment thereof.

23. The nucleic acid agent of claim 22, wherein n is 6 or less.

24. The nucleic acid agent of any one of claims 21 to 23, wherein the repeat sequence comprises a natural ribonucleoside and/or a non-natural ribonucleoside.

25. The nucleic acid agent of claim 24, wherein the non-natural ribonucleoside is 2'-O-methyl-modified nucleoside, 2'-O-methoxyethyl-modified nucleoside, or 2'-O-[2-(N-methylcarbamoyl)ethyl]-modified nucleoside.

26. The nucleic acid agent of claim 1 for reducing aggregation of the TDP-43 protein and/or the fragment thereof.

27. A pharmaceutical composition comprising the nucleic acid agent of claim 1 as an active ingredient.

28. The pharmaceutical composition of claim 27 for preventing or treating a neurodegenerative disease.

29. The pharmaceutical composition of claim 28, wherein the neurodegenerative disease is TDP-43 proteinopathy.

30. The pharmaceutical composition of claim 28 or 29, wherein the neurodegenerative disease is selected from the group consisting of amyotrophic lateral sclerosis which is sporadic (ALS) or familial, frontotemporal lobar degeneration (FTLD), Alzheimer's disease, dementia with Lewy bodies, Huntington's disease, Parkinson's disease, argyrophilic grain dementia (Grene's disease), Perry syndrome, progressive supranuclear palsy, corticobasal degeneration, and multiple system atrophy.
